# EUROPEAN PATENT APPLICATION

(11) **EP 4 332 677 A1**
(43) Date of publication of application: **06.03.2024**
(21) Application number: 23190820.3
(22) Date of filing: 10.08.2023
(51) Int. Cl.: G03F 7/11, G03F 7/038

(54) **PATTERN FORMING METHOD**

(30) Priority: 17.08.2022 JP 2022130260
(71) Applicant: SHIN-ETSU CHEMICAL CO., LTD., Tokyo 1000005 (JP)
(72) Inventor: Tachibana, Seiichiro, Niigata (JP); Watanabe, Takeru, Niigata (JP); Kori, Daisuke, Niigata (JP); Sawamura, Takashi, Niigata (JP)
(74) Representative: Sonnenhauser, Thomas Martin

(57) **Abstract**

The present invention provides a pattern forming method using an adhesion film forming composition comprising (A) a polymer compound, (B) a thermal acid generator, and (C) an organic solvent includes steps of:
(I-1) applying the adhesion film forming composition onto a substrate to be processed and thereafter performing thermal treatment to form an adhesion film;
(I-2) forming a resist upper layer film on the adhesion film using a resist upper layer film forming composition; (I-3) performing pattern exposure on the resist upper layer film and thereafter developing the resist upper layer film with a developer to form a circuit pattern on the resist upper layer film; and (I-4) transferring a pattern to the adhesion film and the substrate to be processed by dry etching with the resist upper layer film on which the circuit pattern is formed as a mask.

## Description

### TECHNICAL FIELD

The present invention relates to a pattern forming method.

### BACKGROUND ART

As large-Scale integrated circuits (LSIs) advance toward high integration and high processing speed, miniaturization of a pattern size is progressing rapidly. Along with the miniaturization, a lithography technology has achieved formation of a fine pattern by shortening a wavelength of a light source and selecting an appropriate resist composition accordingly. The composition mainly used is a positive photoresist composition for a monolayer. The monolayer positive photoresist composition contains a resist resin that has a skeleton having resistance to dry etching with chlorine- or fluorine-based gas plasma, and provides a switching mechanism that makes an exposed part soluble, whereby the exposed part is dissolved to form a pattern and dry etching is performed on a substrate to be processed to which the photoresist composition is applied with the remaining resist pattern as an etching mask.

However, when the pattern becomes finer, that is, the pattern width is reduced without changing the thickness of a photoresist film to be used, resolution performance of the photoresist film is lowered. In addition, development of the photoresist film using a chemical excessively increases an aspect ratio of a line pattern in a line-and-space pattern, resulting in collapse of a pattern. Thus, the photoresist film has been thinned along with the miniaturization of the pattern.

On the other hand, the substrate to be processed has been generally processed by a method of processing the substrate to be processed by dry etching using a pattern-formed photoresist film as an etching mask. However, since the photoresist film has been thinned, etching selectivity between the photoresist film and the substrate to be processed cannot be ensured. That is, there is an issue that the photoresist film is damaged and collapses during processing of the substrate to be processed so that the resist pattern cannot be accurately transferred to the substrate to be processed. Accordingly, higher dry etching resistance has been required in the photoresist composition along with the miniaturization of the pattern. Meanwhile, a resin used for the photoresist composition has been required to have low light absorption at an exposure wavelength. The resin used for the photoresist composition thus shifts in structure to a novolak resin, polyhydroxystyrene, and a resin having an aliphatic polycyclic skeleton as the exposure light shifts from i-line to KrF and ArF, which have a shorter wavelength. This means acceleration of an etching rate under the above-mentioned dry etching conditions. That is, recent photoresist compositions having high resolution tend to have lower etching resistance.

Thus, the substrate to be processed has to be subjected to dry etching with a thinner photoresist film having lower etching resistance, and there is a demand for a material and a process in this work process.

A multilayer resist method is one of solutions for these issues. This method is as follows: a resist middle layer film having different etching selectivity from a photoresist film (i.e., a resist upper layer film) is placed between the resist upper layer film and a substrate to be processed; a pattern is formed in the resist upper layer film; the pattern is transferred to the resist middle layer film by dry etching using the resist upper layer film pattern as a dry etching mask; the pattern is further transferred to the substrate to be processed by dry etching using the resist middle layer film as a dry etching mask.

Examples of the multilayer resist method include a three-layer resist method, which can be employed with a typical resist composition used in a monolayer resist method. For example, this three-layer resist method includes the following steps: forming an organic film containing novolak or the like as a resist underlayer film on a substrate to be processed; forming a silicon-containing film thereon as a silicon-containing resist middle layer film; and forming a usual organic photoresist film thereon as a resist upper layer film. Since the organic resist upper layer film exhibits a favorable etching selectivity ratio relative to the silicon-containing resist middle layer film when dry etching is performed with fluorine-based gas plasma, the resist upper layer film pattern can be transferred to the silicon-containing resist middle layer film by dry etching with fluorine-based gas plasma. Since the silicon-containing resist middle layer exhibits a favorable etching selectivity ratio relative to the resist underlayer film when etching is performed with oxygen gas or hydrogen gas, the silicon-containing resist middle layer film can be transferred to the resist underlayer film by etching with oxygen gas or hydrogen gas. According to this method, if the pattern can be transferred to the silicon-containing film (the silicon-containing resist middle layer film) using a photoresist composition that is difficult to form a pattern having a sufficient film thickness for directly processing the substrate to be processed or a photoresist composition that has insufficient dry etching resistance for processing the substrate, it is possible to obtain a pattern of an organic film (resist underlayer film) made of novolak or the like, which has sufficient dry etching resistance for processing.

In recent years, as a potential technique for combined use of ArF immersion lithography and a multi-exposure process, attention has been drawn to vacuum ultraviolet light (extreme-ultraviolet (EUV)) lithography with a wavelength of 13.5 nm. With use of this technique, it becomes possible to form a fine pattern at a half pitch of 25 nm or less by one exposure.

Meanwhile, in the EUV lithography, improvement of sensitivity is strongly required for a resist material to supplement output shortage of a light source. However, increased shot noise along with improvement of sensitivity leads to increased edge roughness of a line pattern (line edge roughness (LER) and line width roughness (LWR)). Achieving both improvement of sensitivity and low edge roughness is cited as one of important issues in the EUV lithography.

In an attempt to improve sensitivity of a resist and reduce influence of shot noise, use of a metal material as a resist material has been examined in recent years. A compound containing a metal element such as barium, titanium, hafnium, zirconium, and tin has higher absorbance of EUV light than that of an organic material that does not contain metal, and can be expected to improve photosensitivity of a resist and suppress influence of shot noise. In addition, it is expected that a metal-containing resist pattern in combination with an underlayer film made of a non-metallic material can be subjected to etching with a higher etching selectivity ratio.

For example, resist materials using metallic salts, an organometallic complex, and a metallic cluster (Patent Documents 1 to 5, and Non-Patent Document 1) have been examined. However, resolution of these metal-containing resists has not reached a level that is necessary for practical use, and further improvement of resolution is required.

Furthermore, with appearance of ArF immersion lithography and EUV lithography, it has become possible to form a finer pattern. Meanwhile, an ultrafine pattern has a small ground area and is highly likely to collapse, and it is an enormous issue to prevent collapse of a pattern. To prevent collapse of the pattern, reported are materials that use a resist underlayer film containing a polar functional group such as a lactone structure and an urea structure to improve adhesiveness with respect to a resist upper layer film (Patent Documents 6 and 7). However, under the current circumstances where formation of finer patterns is required, it cannot be said that these materials have sufficient performance in preventing collapse of the pattern.

In recent years, interaction in an interface between a resist upper layer film and a resist underlayer film in a fine pattern is considered to affect sensitivity of the resist and a pattern shape (rectangularity and a residue in a space), and improvement of performance of the resist underlayer film is required also from this perspective (Non-Patent Document 2). Consequently, in leading end microfabrication, there has been a demand for an adhesion film that does not generate a residue in a space (portion where the resist upper layer film is removed by development) in a pattern, that allows for patterning with high rectangularity, and furthermore, that prevents collapse of the pattern.

### CITATION LIST

### PATENT LITERATURE

Patent Document 1: JP 5708521 B2
Patent Document 2: JP 5708522 B2
Patent Document 3: JP 2021-033090 A
Patent Document 4: JP 2021-039171 A
Patent Document 5: US 9310684 B2
Patent Document 6: WO 2003/017002 A1
Patent Document 7: WO 2018/143359 A1
Patent Document 8: JP 6404757 B2

### NON PATENT LITERATURE

Non Patent Document 1: Proc. SPIE Vol. 7969, 796915 (2011)
Non Patent Document 2: Proc. SPIE Vol. 7273, 72731J (2009)

### SUMMARY OF INVENTION

### TECHNICAL PROBLEM

The present invention has been made in consideration of the above-mentioned circumstances, and aims to provide a pattern forming method that allows for obtaining of a favorable pattern shape and that uses an adhesion film having high adhesiveness with respect to a resist upper layer film and preventing collapse of a fine pattern, in a fine patterning process in a manufacturing process for a semiconductor apparatus.

### SOLUTION TO PROBLEM

To solve the above-mentioned problem, the present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(I-1) applying the adhesion film forming composition onto the substrate to be processed and thereafter performing thermal treatment to form an adhesion film;
(I-2) forming a resist upper layer film on the adhesion film using a resist upper layer film forming composition;
(I-3) performing pattern exposure on the resist upper layer film and thereafter developing the resist upper layer film with a developer to form a circuit pattern on the resist upper layer film; and
(I-4) transferring a pattern to the adhesion film and the substrate to be processed by dry etching with the resist upper layer film on which the circuit pattern is formed as a mask, 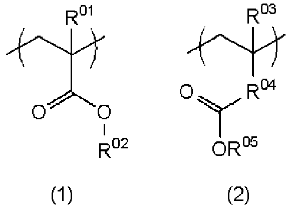 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 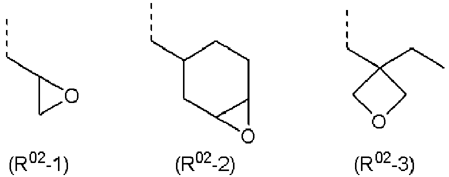 wherein a broken line represents a bond.

The present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(II-1) forming a resist underlayer film on the substrate to be processed;
(II-2) forming a silicon-containing resist middle layer film on the resist underlayer film;
(II-3) applying the adhesion film forming composition onto the silicon-containing resist middle layer film and thereafter performing thermal treatment to form an adhesion film;
(II-4) forming a resist upper layer film on the adhesion film using a resist upper layer film forming composition;
(II-5) performing pattern exposure on the resist upper layer film and thereafter developing the resist upper layer film with a developer to form a circuit pattern on the resist upper layer film;
(II-6) transferring a pattern to the adhesion film and the silicon-containing resist middle layer film by dry etching with the resist upper layer film on which the circuit pattern is formed as a mask;
(II-7) transferring a pattern to the resist underlayer film by dry etching with the silicon-containing resist middle layer film to which the pattern is transferred as a mask; and
(II-8) processing the substrate to be processed to form a pattern on the substrate to be processed with the resist underlayer film to which the pattern is transferred as a mask, 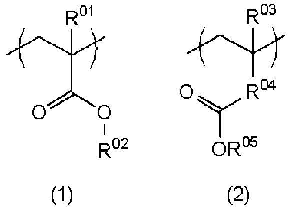 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 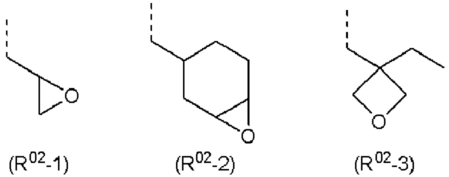 wherein a broken line represents a bond.

The present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(III-1) forming a resist underlayer film on the substrate to be processed;
(III-2) forming, on the resist underlayer film, an inorganic hard mask middle layer film selected from the group consisting of a silicon oxide film, a silicon nitride film, and a silicon oxide nitride film;
(III-3) applying the adhesion film forming composition onto the inorganic hard mask middle layer film and thereafter performing thermal treatment to form an adhesion film;
(III-4) forming a resist upper layer film on the adhesion film using a resist upper layer film forming composition;
(III-5) performing pattern exposure on the resist upper layer film and thereafter developing the resist upper layer film with a developer to form a circuit pattern on the resist upper layer film;
(III-6) transferring a pattern to the adhesion film and the inorganic hard mask middle layer film by dry etching with the resist upper layer film on which the circuit pattern is formed as a mask;
(III-7) transferring a pattern to the resist underlayer film by dry etching with the inorganic hard mask middle layer film to which the pattern is transferred as a mask; and
(III-8) processing the substrate to be processed to form a pattern on the substrate to be processed with the resist underlayer film to which the pattern is transferred as a mask, 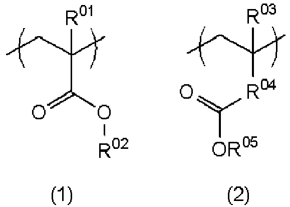 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 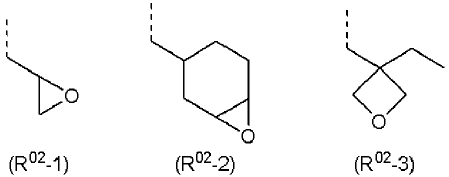 wherein a broken line represents a bond.

In this manner, the pattern forming method according to the present invention is implemented by various kinds of pattern forming methods such as a two-layer resist process and a four-layer resist process in which the above-mentioned adhesion film is formed on a silicon-containing middle layer film (a silicon-containing resist middle layer film and an inorganic hard mask middle layer film). These pattern forming methods can effectively prevent collapse of the pattern using the adhesion film, and are preferable for photolithography on the resist upper layer film.

At this time, the inorganic hard mask middle layer film is preferably formed by a carbon vapor deposition (CVD) method or an atomic layer deposition (ALD) method.

With use of the above-mentioned pattern forming method, the present invention enables effective pattern formation.

In the pattern forming method according to the present invention, a film thickness of the adhesion film immediately below the resist upper layer film is preferably 15 nm or less.

Such a film thickness can decrease load on a resist pattern in an etching process, and thus is preferable.

The resist upper layer film used in the present invention may be formed using the resist upper layer film forming composition comprising at least a metallic atom-containing compound and an organic solvent.

In this case, the metallic atom-containing compound preferably comprises at least one selected from the group consisting of titanium, cobalt, copper, zinc, zirconium, lead, indium, tin, antimony, and hafnium.

With use of the resist upper layer film in the pattern forming method according to the present invention, collapse of a fine pattern is prevented, and a favorable pattern shape can be obtained. At the same time, contamination of the substrate to be processed by a metallic compound can be prevented.

In the present invention, as a method for forming a circuit pattern on the resist upper layer film, photolithography with a wavelength of 10 nm or more and 300 nm or less, electron beam direct drawing, nanoimprinting, or a combination thereof is preferably used.

In the present invention, as a development method, alkaline development or development with an organic solvent is preferably used.

In the present invention, pattern formation can be favorably and effectively performed also with use of the above-mentioned pattern forming method.

In the present invention, as the substrate to be processed, a semiconductor apparatus substrate, or the semiconductor apparatus substrate on which any one of a metal film, a metal carbide film, a metal oxide film, a metal nitride film, a metal oxide carbide film, and a metal oxide nitride film is formed is preferably used.

At this time, as the metal, silicon, titanium, tungsten, hafnium, zirconium, chrome, germanium, copper, silver, gold, aluminum, indium, gallium, arsenic, palladium, iron, tantalum, iridium, cobalt, manganese, molybdenum, or an alloy thereof is preferably used.

With the pattern forming method according to the present invention, the above-mentioned substrate to be processed is processed as just described and the pattern can be formed.

In the pattern forming method according to the present invention, the adhesion film can be formed by rotary application of the adhesion film forming composition, and thermal treatment on a substrate to which the adhesion film forming composition is applied at a temperature of 100°C or more and 300°C or less during a period of time in a range of 10 to 600 seconds.

In the pattern forming method according to the present invention, the adhesion film can be formed by rotary application of the adhesion film forming composition, and thermal treatment on a substrate to which the adhesion film forming composition is applied in an atmosphere with an oxygen concentration of 0.1% or more and 21% or less.

Such a method can promote cross-linking reaction at the time of formation of the adhesion film and further prevent mixing with the resist upper layer film. Adjusting a thermal treatment temperature, time, or an oxygen concentration within the above-mentioned ranges can provide an effect of preventing collapse of the resist pattern on the adhesion film and adjust the pattern shape.

In the pattern forming method according to the present invention, the adhesion film can be formed by rotary application of the adhesion film forming composition, and thermal treatment on a substrate to which the adhesion film forming composition is applied in an atmosphere with an oxygen concentration of less than 0.1%.

Such a method can promote cross-linking reaction at the time of formation of the adhesion film and prevent inter-mixing with the upper layer film without degradation of the substrate to be processed even in a case where the substrate to be processed includes a material that is unstable to heating under an oxygen atmosphere, and thus is advantageous.

### ADVANTAGEOUS EFFECTS OF INVENTION

As described above, in the present invention, with the introduction of the adhesion film, it is possible to form the resist upper layer film pattern having higher rectangularity without collapse, and with a multilayer resist process such as the four-layer resist process to form the adhesion film on the silicon-containing middle layer film or the inorganic hard mask middle layer film, it is possible to form the fine pattern on the substrate to be processed.

### BRIEF DESCRIPTION OF DRAWINGS

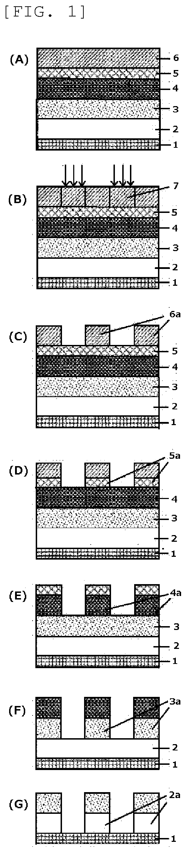

FIG. 1 is explanatory view showing an example of a pattern forming method using a four-layer resist process according to the present invention.

### DESCRIPTION OF EMBODIMENTS

In the present description, when an element is "immediately below" another element, the element is in direct contact with the other element without any intervening element therebetween. In contrast, when an element is "below" another element, an intervening element can exist therebetween. Similarly, when an element is "immediately above" another element, the element is in direct contact with the other element without any intervening element therebetween, and when an element is "above" another element, an intervening element can exist therebetween.

As described above, there has been a demand for development of the pattern forming method that allows for obtaining of a favorable pattern shape, and that uses the adhesion film having adhesiveness with respect to the resist upper layer film and preventing collapse of a fine pattern, in the fine patterning process in the manufacturing process for a semiconductor apparatus.

As a result of earnest examination of the above-mentioned issues, the present inventors have found that a pattern forming method using an adhesion film formed of the following adhesion film forming composition can solve the above-mentioned issues and have completed the present invention.

That is, the present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(I-1) applying the adhesion film forming composition onto the substrate to be processed and thereafter performing thermal treatment to form an adhesion film;
(I-2) forming a resist upper layer film on the adhesion film using a resist upper layer film forming composition;
(I-3) performing pattern exposure on the resist upper layer film and thereafter developing the resist upper layer film with a developer to form a circuit pattern on the resist upper layer film; and
(I-4) transferring a pattern to the adhesion film and the substrate to be processed by dry etching with the resist upper layer film on which the circuit pattern is formed as a mask, 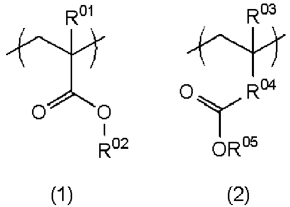 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 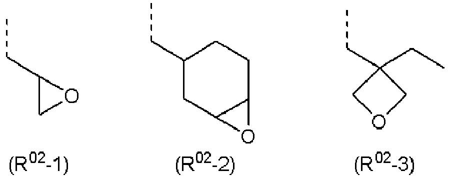 wherein a broken line represents a bond.

In addition, the present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(II-1) forming a resist underlayer film on the substrate to be processed;
(II-2) forming a silicon-containing resist middle layer film on the resist underlayer film;
(II-3) applying the adhesion film forming composition onto the silicon-containing resist middle layer film and thereafter performing thermal treatment to form an adhesion film;
(II-4) forming a resist upper layer film on the adhesion film using a resist upper layer film forming composition;
(II-5) performing pattern exposure on the resist upper layer film and thereafter developing the resist upper layer film with a developer to form a circuit pattern on the resist upper layer film;
(II-6) transferring a pattern to the adhesion film and the silicon-containing resist middle layer film by dry etching with the resist upper layer film on which the circuit pattern is formed as a mask;
(II-7) transferring a pattern to the resist underlayer film by dry etching with the silicon-containing resist middle layer film to which the pattern is transferred as a mask; and
(II-8) processing the substrate to be processed to form a pattern on the substrate to be processed with the resist underlayer film to which the pattern is transferred as a mask, 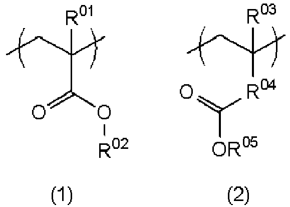 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 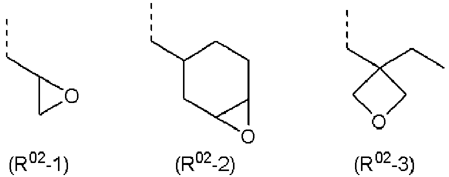 wherein a broken line represents a bond.

Furthermore, the present invention provides a pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(III-1) forming a resist underlayer film on the substrate to be processed;
(III-2) forming, on the resist underlayer film, an inorganic hard mask middle layer film selected from the group consisting of a silicon oxide film, a silicon nitride film, and a silicon oxide nitride film;
(III-3) applying the adhesion film forming composition onto the inorganic hard mask middle layer film and thereafter performing thermal treatment to form an adhesion film;
(III-4) forming a resist upper layer film on the adhesion film using a resist upper layer film forming composition;
(III-5) performing pattern exposure on the resist upper layer film and thereafter developing the resist upper layer film with a developer to form a circuit pattern on the resist upper layer film;
(III-6) transferring a pattern to the adhesion film and the inorganic hard mask middle layer film by dry etching with the resist upper layer film on which the circuit pattern is formed as a mask;
(III-7) transferring a pattern to the resist underlayer film by dry etching with the inorganic hard mask middle layer film to which the pattern is transferred as a mask; and
(III-8) processing the substrate to be processed to form a pattern on the substrate to be processed with the resist underlayer film to which the pattern is transferred as a mask, 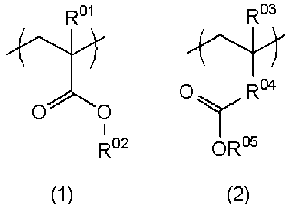 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 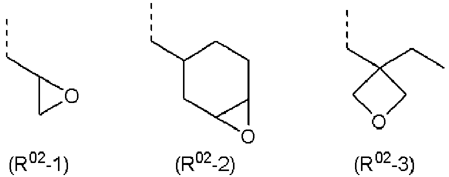 wherein a broken line represents a bond.

The present invention will be described in detail below, but is not limited to what is described.

### Adhesion film Forming Composition

An adhesion film forming composition for forming an adhesion film immediately below a resist upper layer film contains (A) a polymer compound containing a repeating unit shown by the following general formula (1) and a repeating unit shown by the following general formula (2), (B) a thermal acid generator, and (C) an organic solvent. 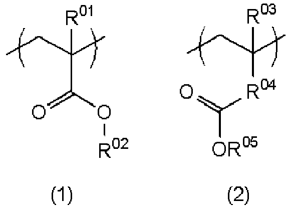 (In the above-mentioned formulae, R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and may further contain an oxygen functional group.) 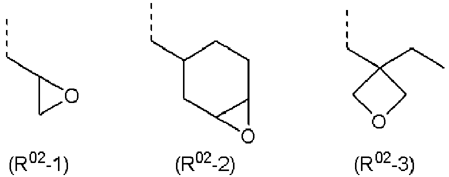 (In the above-mentioned formulae, a broken line represents a bond.)

Note that in the adhesion film forming composition used in the present invention, one kind of the polymer compound (A) can be used alone, or two or more kinds thereof can be used in combination. Furthermore, the above-mentioned adhesion film forming composition may contain a component other than the above-mentioned components (A) to (C). Each component will be described below.

### (A) Polymer Compound

The polymer compound (A) contained in the adhesion film forming composition contains the repeating unit shown by the following general formula (1) and the repeating unit shown by the following general formula (2) . 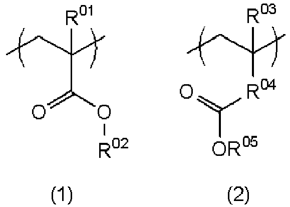 (In the formulae, R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and may further contain an oxygen functional group.) 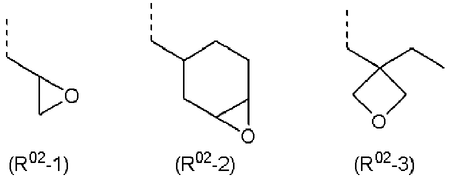 (In the above-mentioned formulae, a broken line represents a bond.)

Specific, preferable examples of the repeating unit shown by the above-mentioned general formula (1) can include the following structure. R⁰¹ independently represents a hydrogen atom or a methyl group.

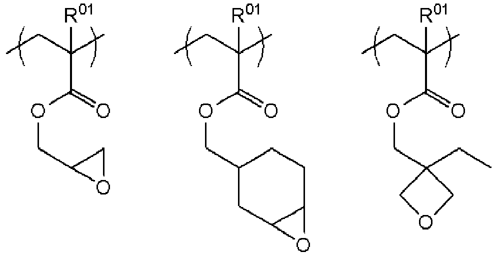

The repeating unit shown by the above-mentioned general formula (1) functions as a cross-linking unit, and an epoxy structure or an oxetane structure causes ring-opening reaction at the time of film formation by heating, resulting in curing of a film. As described above, combined use of the repeating unit shown by the above-mentioned general formula (1) in the polymer compound is effective for curing of the film and enables intricate film formation. A hydroxy group that is generated by the ring-opening reaction also contributes to improvement of adhesiveness with respect to the resist upper layer film.

In the repeating unit shown by the above-mentioned general formula (2), R⁰³ independently represents a hydrogen atom or a methyl group.

R⁰⁴ represents a single bond or a divalent linking group containing an ester group and having 2 to 10 carbon atoms. Specific examples can include a single bond, -CO₂CH₂-, -CO₂CH₂CH₂-, -CO₂CH₂CH₂CH₂-, -CO₂CH (CH₃) -, - CO₂CH₂CH₂CH₂CH₂-, -CO₂CH₂CH₂CH₂CH₂CH₂-, - CO₂CH₂CH₂CH₂CH₂CH₂CH₂CH₂CH₂-, -CO₂CH₂CH₂O-, - CO₂CH₂CH₂OCH₂OCH₂O-, and -CO₂CH₂CH₂OCH₂CH₂OCH₂CH₂O-. Among them, -CO₂CH₂-, -CO₂CH₂CH₂-, -CO₂CH₂CH₂CH₂-, - CO₂CH₂CH₂CH₂CH₂-, and -CO₂CH₂CH₂CH₂CH₂CH₂-, are particularly preferable.

R⁰⁵ represents a saturated or unsaturated tertiary alkyl group having 4 to 20 carbon atoms, and may further contain an oxygen functional group. Examples of R⁰⁵ can include the following structures, but R⁰⁵ is not limited thereto.

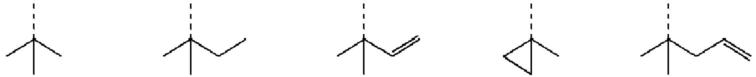

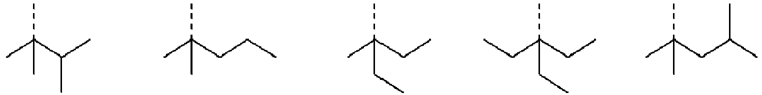

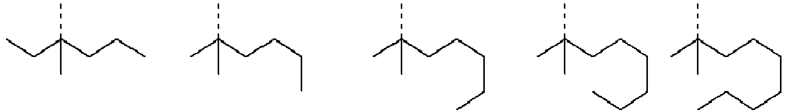

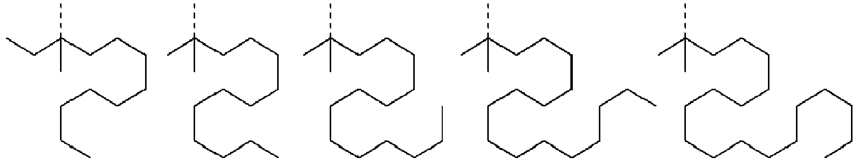

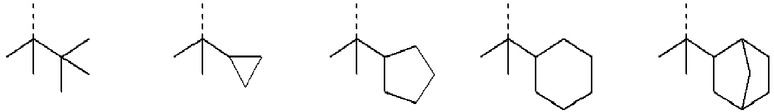

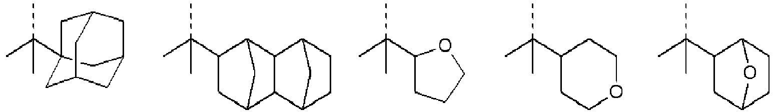

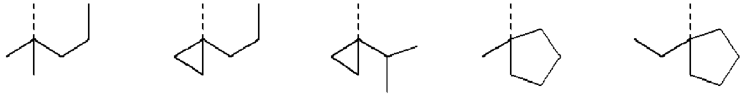

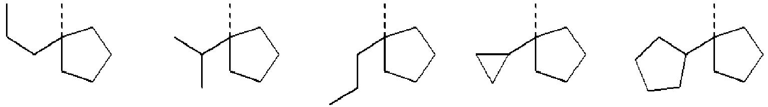

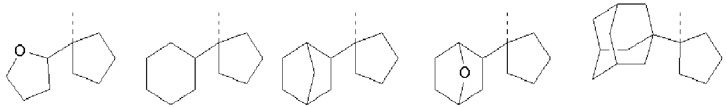

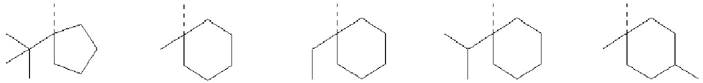

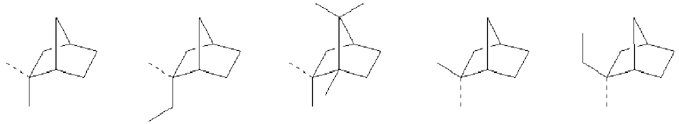

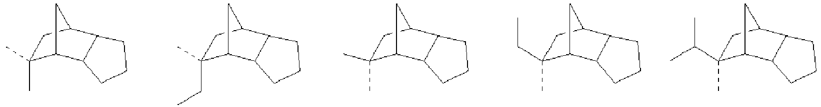

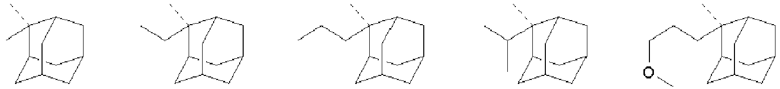

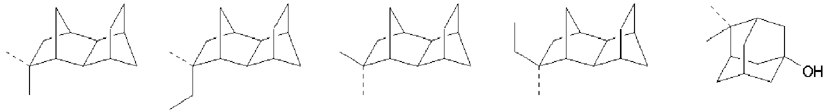

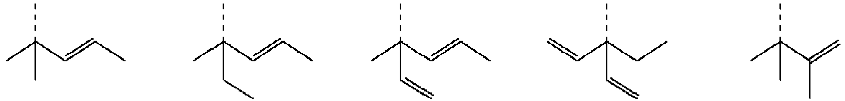

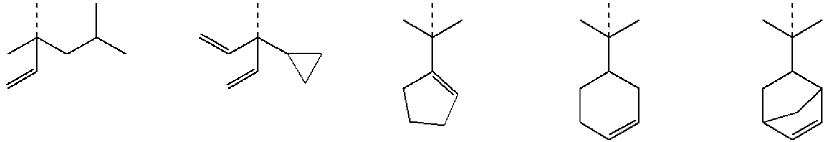

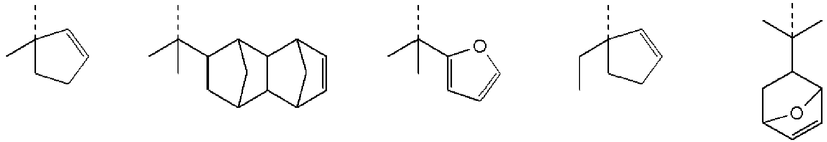

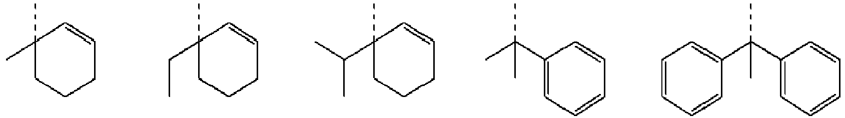

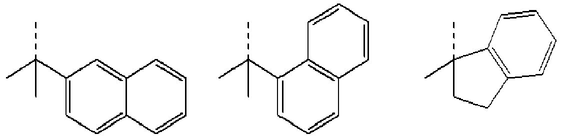

In a case of forming the adhesion film from the polymer compound containing the repeating unit shown by the above-mentioned general formula (2), desorption/decomposition reaction of the tertiary alkyl group R⁰⁵ advances due to heat and/or action of acid generated by an acid generator, which will be described later, and carboxylic acid is generated. There is a case where adhesiveness with respect to the resist pattern improves, rectangularity of the pattern improves, and generation of a residue in a space in the pattern is prevented depending on polarity of the carboxylic acid.

Additionally, there is also a case where the generated carboxylic acid causes ring-opening addition reaction with the repeating unit shown by the general formula (1) to form a hydroxy ester cross-linking structure. A typical example of reaction is described below. In the following formulae, (3) shows a state where R⁰⁵ in the repeating unit shown by (2) is desorbed and (2) becomes carboxylic acid, and (4) is a state where ring-opening addition reaction occurs between (3) and (1"), which is an example of the above-mentioned general formula (1), and a hydroxy ester cross-linking structure is formed. A portion surrounded by a dotted line is the hydroxy ester cross-linking structure formed by this reaction. 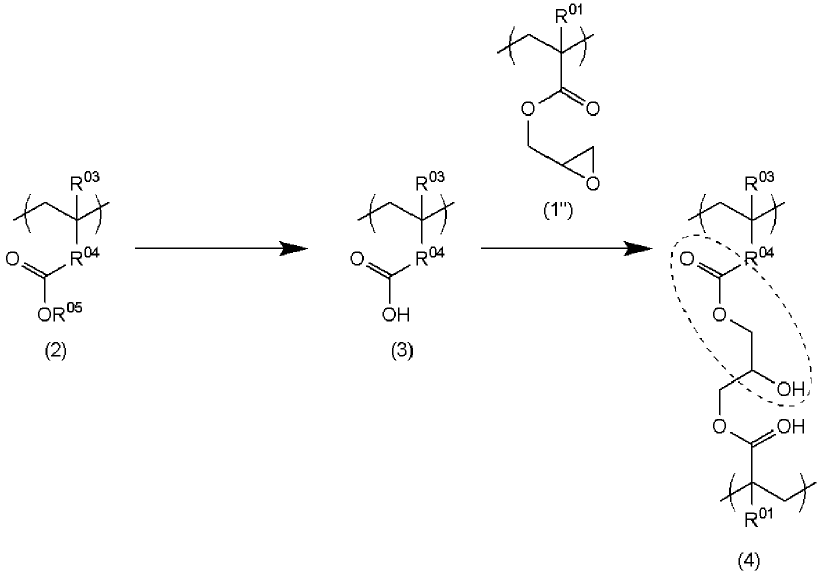 (In the formulae, R⁰¹, R⁰³, R⁰⁴, and R⁰⁵ are as described above.)

Formation of the hydroxy ester cross-linking structure is cross-linking reaction, and promotes curing of the adhesion film. If the effect is sufficient, an intricate film is formed, and inter-mixing between the adhesion film and the resist upper layer film is prevented, whereby a residue in a space in the pattern can be prevented, and the pattern having high rectangularity can be obtained.

Since the above-mentioned hydroxy ester cross-linking structure is a polar group and interacts with the resist pattern, the presence of the repeating unit (2) contributes also to prevention of collapse of the resist pattern.

Note that one kind of the repeating unit shown by the above-mentioned general formula (2) may be contained in the polymer compound (A) or two or more kinds thereof may be contained in the polymer compound (A).

It is preferable that in the above-mentioned polymer compound (A), a content rate of the repeating unit shown by the above-mentioned general formula (1) be 10 mol% or more and 90 mol% or less with respect to the entire repeating units, and a content rate of the repeating unit shown by the above-mentioned general formula (2) be 10 mol% or more and 90 mol% or less with respect to the entire repeating units.

By setting such content rates of the above-mentioned general formulae (1) and (2), the polarity of the polymer compound (A) is adjusted, and the adhesiveness with respect to the resist pattern becomes favorable. In addition, at the time of film formation by heating, the intricate adhesion film can be formed by reaction between carboxylic acid generated by the above-mentioned general formula (2) and the above-mentioned general formula (1), and inter-mixing between the adhesion film and the resist upper layer film can be prevented. This can prevent generation of a residue in a space in the pattern, and enables obtaining of the pattern having high rectangularity. Thus, in the above-mentioned polymer compound (A), it is preferable that a content rate of the repeating unit shown by the above-mentioned general formula (1) be 10 mol% or more and 90 mol% or less, particularly 20 mol% or more and 80 mol% or less with respect to the entire repeating units, and a content rate of the repeating unit shown by the above-mentioned general formula (2) be 10 mol% or more and 90 mol% or less, particularly 20 mol% or more and 80 mol% or less with respect to the entire repeating units.

It is preferable that the polymer compound (A) further contain a repeating unit shown by the following general formula (5). 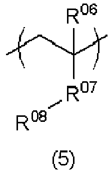 (In the formula, R⁰⁶ represents a hydrogen atom or a methyl group; R⁰⁷ represents a single bond, an ester group, or a divalent linking group containing an ester group and having 2 to 10 carbon atoms; and R⁰⁸ represents a substituted or unsubstituted aryl group having 6 to 20 carbon atoms.)

In the above-mentioned general formula (5), R⁰⁶ represents a hydrogen atom or a methyl group. R⁰⁷ represents a single bond, an ester group (-CO₂-), or a divalent linking group containing an ester group and having 2 to 10 carbon atoms. Specific examples can include a single bond, -CO₂-, -CO₂CH₂-, -CO₂CH₂CH₂-, - CO₂CH₂CH₂CH₂-, -CO₂CH (CH₃) -, -CO₂CH₂CH₂CH₂CH₂-, - CO₂CH₂CH₂CH₂CH₂CH₂CH₂CH₂CH₂-, -CO₂CH₂CH₂O-, - CO₂CH₂CH₂OCH₂CH₂O-, and -CO₂CH₂CH₂OCH₂CH₂OCH₂CH₂O-, but R⁰⁷ is not limited thereto.

R⁰⁸ represents a substituted or unsubstituted aryl group having 6 to 20 carbon atoms. Specific examples can include a phenyl group, a tolyl group, a xylyl group, a methoxyphenyl group, a tert-butoxyphenyl group, a hydroxyphenyl group, an acetylphenyl group, a naphthyl group, a methyl naphthyl group, an anthracenyl group, a phenanthrenyl group, and a pyrenyl group. The phenyl group and the tert-butoxyphenyl group are particularly preferable. However, R⁰⁸ is not limited thereto.

Note that, in a case where the polymer compound (A) contains the repeating unit (5), one kind of the repeating unit (5) may be contained or two or more kinds thereof may be contained. In a case where the polymer compound (A) contains the repeating unit (5), it is preferable that a content rate of the repeating unit (5) be 5 mol% or more and 50 mol% or less, particularly 5 mol% or more and 40 mol% or less with respect to the entire repeating units. The content rate of the repeating unit (5) being 5 mol% or more is preferable because resolution at the time of lithography on the resist upper layer film becomes favorable. The content rate of the repeating unit (5) being 50 mol% or less is preferable because etching characteristics becomes favorable.

A weight-average molecular weight of the above-mentioned polymer compound (A) is preferably 8,000 to 50,000, and a degree of dispersion defined by the weight-average molecular weight/a number average molecular weight is preferably 3.0 or less.

Note that the "weight-average molecular weight" mentioned herein is a value measured by gel permeation chromatography (GPC) using tetrahydrofuran as a solvent and polystyrene as a standard substance. By setting the weight-average molecular weight and the degree of dispersion of the polymer compound (A) contained in the adhesion film forming composition within such ranges, an excellent film-forming property can be obtained at the time of spin-coating, generation of a sublimate can be prevented at the time of curing by heating, whereby contamination of an apparatus can be prevented. Particularly in formation of a thin film with a thickness of 15 nm or less, when a volatile component having a low molecular weight is contained in a composition, distribution of film thicknesses within a wafer plane tends to vary. However, by setting the molecular weight and degree of dispersion of the polymer compound to be used as described above and managing an amount of a low molecular weight component, the distribution of film thicknesses within the wafer plane can be made small. Thus, the weight-average molecular weight of the polymer compound (A) is preferably 8,000 to 50,000. The degree of dispersion is preferably 3.0 or less.

Examples of a method of synthesizing the polymer compound (A) include a method of blending a monomer having a polymerizable unsaturated bond corresponding to each repeating unit and adding a radical polymerization initiator to a solvent to perform polymerization by heating. Polymerization conditions can be selected in various manners depending on a monomer to be used, a target molecular weight, and the like, and are not particularly limited. Specific examples of the polymerization conditions can include toluene, benzene, tetrahydrofuran, diethyl ether, dioxane, 2-butanone, methyl isobutyl ketone, propyleneglycol monomethylether acetate, cyclohexanone, γ-butyrolactone, ethyl acetate, butyl acetate, and diacetone alcohol. Examples of the radical polymerization initiator can include 2,2'-azobisisobutyronitrile (AIBN), 2,2'-azobis(2,4-dimethylvaleronitrile), dimethyl 2,2-azobis(2-methylpropionate), benzoyl peroxide, and lauroyl peroxide. In addition, as a chain transfer agent, thiols such as octanethiol and 2-mercaptoethanol may be added at the time of polymerization. Polymerization reaction can be preferably caused by heating from 40°C to a boiling point of a reaction solvent. Reaction time is preferably 0.5 to 100 hours, more preferably, 1 to 48 hours.

For example, the polymer compound containing the repeating units shown by the above-mentioned formulae (1), (2), and (5) can be synthesized by the above-mentioned polymerization using compounds having polymerizable double bonds shown by the following general formulae (1'), (2'), and (5') as monomers. 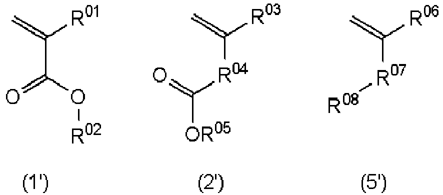 (In the formulae, R⁰¹ to R⁰⁸ are as described above.)

In polymerization, heating may be performed after all raw materials are blended, or part of the raw materials is preliminarily heated and the remaining raw materials are added in the part of the raw materials at once or by gradation on an individual basis or in a blended manner. For example, a method of preliminarily heating only a polymerization solvent, and separately, gradually adding a monomer solution and a polymerization initiator solution to the heated polymerization solvent enables obtaining of a relatively homogeneous polymer compound, and can prevent abnormal reaction such as runaway reaction, and thus is particularly preferable.

The polymer compound solution obtained as described above may be blended in the adhesion film forming composition as it is, or may be purified using a routine method such as crystallization, liquid separation, filtration, and condensation so that residual monomers, a residual solvent, reaction by-products, other impurities, and the like may be removed. In a case of purifying the polymer compound (A), a crystallization method of adding a poor solvent such as water, hydroalcohol, and saturated hydrocarbon to the polymer compound solution and collecting a resulting precipitate by filtration, or a liquid separation method of separating and removing a poor solvent layer is preferable. Among these methods, the liquid separation method is particularly preferable. When the polymer compound is purified by the liquid separation method, low molecular weight components in the polymer compound solution can be efficiently removed, so that the sublimate generation when the adhesion film is formed from the adhesion film forming composition containing the polymer compound is suppressed, and consequently, contamination of a film-forming apparatus can be prevented.

### (B) Thermal Acid Generator

The thermal acid generator (B) is added to the adhesion film forming composition used in the present invention to promote cross-linking reaction by heat.

Examples of the thermal acid generator (B) that can be used in the adhesion film forming composition used in the present invention include the following general formula (6). 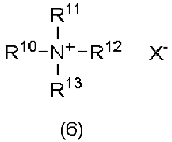 (In the formula, X⁻ represents a non-nucleophilic counter ion. R¹⁰, R¹¹, R¹², and R¹³ each represent a hydrogen atom, or a linear, branched, or cyclic alkyl group having 1 to 12 carbon atoms, an alkenyl group, an oxoalkyl group, or an oxoalkenyl group, an aryl group having 6 to 20 carbon atoms, an aralkyl group having 7 to 12 carbon atoms, or an aryloxoalkyl group, and part or all of hydrogen atoms of these groups may be substituted with alkoxy groups or the like. R¹⁰ and R¹¹, or R¹⁰, R¹¹, and R¹² may form a ring. In a case of forming the ring, R¹⁰ and R¹¹, or R¹⁰, R¹¹, and R¹² represent an alkylene group having 3 to 10 carbon atoms, or a heteroaromatic ring having a nitrogen atom in the formula (3) within the ring.)

The above-mentioned R¹⁰, R¹¹, R¹², and R¹³ may be identical or different from each other. Specific examples of the alkyl group include a methyl group, an ethyl group, a propyl group, an isopropyl group, an n-butyl group, a sec-butyl group, a tert-butyl group, a pentyl group, a hexyl group, a heptyl group, an octyl group, a cyclopentyl group, a cyclohexyl group, a cycloheptyl group, a cyclopropylmethyl group, a 4-methylcyclohexyl group, a cyclohexylmethyl group, a norbornyl group, and an adamantyl group.

Examples of the alkenyl group include a vinyl group, an allyl group, a propenyl group, a butenyl group, a hexenyl group, and a cyclohexenyl group.

Examples of the oxoalkyl group can include a 2-oxocyclopentyl group, a 2-oxocyclohexyl group, a 2-oxopropyl group, a 2-cyclopentyl-2-oxoethyl group, 2-cyclohexyl-2-oxoethyl group, and 2-(4-methylcyclohexyl)-2-oxoethyl group.

Examples of the oxoalkenyl group can include a 2-oxo-4-cyclohexenyl group and a 2-oxo-4-propenyl group.

Examples of the aryl group include: a phenyl group; a naphthyl group; an alkoxyphenyl group such as a p-methoxyphenyl group, a m-methoxyphenyl group, an o-methoxyphenyl, an ethoxyphenyl group, a p-tert-butoxyphenyl group, and an m-tert-butoxyphenyl group; an alkylphenyl group such as a 2-methylphenyl group, a 3-methylphenyl group, a 4-methylphenyl group, an ethylphenyl group, a 4-tert-butylphenyl group, a 4-butylphenyl group, and a dimethylphenyl group; an alkyl naphthyl group such as a methyl naphthyl group and an ethyl naphthyl group; alkoxynaphthyl group such as a methoxynaphthyl group and an ethoxynaphthyl group; a dialkylnaphthyl group such as a dimethylnaphthyl group and a diethylnaphthyl group; a dialkoxynaphthyl group such as a dimethoxynaphthyl group and a diethoxynaphthyl group.

Examples of the aralkyl group include a benzyl group, a phenylethyl group, and a phenethyl group.

Example of the aryloxoalkyl group include a 2-aryl-2-oxoethyl group such as a 2-phenyl-2-oxoethyl group, a 2-(1-naphthyl)-2-oxoethyl group, and a 2-(2-naphthyl)-2-oxoethyl group.

When R¹⁰, R¹¹, R¹², and R¹³ form the heteroaromatic ring having the nitrogen atom in the formula within the ring, examples of R¹⁰, R¹¹, R¹², and R¹³ include an imidazole derivative (for example, imidazole, 4-methylimidazole, 4-methyl-2-phenylimidazole, or the like), a pyrazole derivative, a furazan derivative, a pyrroline derivative (for example, pyrroline, 2-methyl-1-pyrroline, or the like), a pyrrolidine derivative (such as pyrrolidine, N-methylpyrrolidine, pyrrolidinone, N-methylpyrrolidone, or the like), an imidazoline derivative, an imidazolidine derivative, a pyridine derivative (for example, pyridine, methylpyridine, ethylpyridine, propylpyridine, butylpyridine, 4-(1-butylpentyl)pyridine, dimethylpyridine, trimethylpyridine, triethylpyridine, phenylpyridine, 3-methyl-2-phenylpyridine, 4-tert-butylpyridine, diphenylpyridine, benzylpyridine, methoxypyridine, butoxypyridine, dimethoxypyridin, 1-methyl-2-pyridone, 4-pyrrolidinopyridine, 1-methyl-4-phenylpyridine, 2-(1-ethylpropyl)pyridine, aminopyridine, dimethylaminopyridine, or the like), a pyridazine derivative, a pyrimidine derivative, a pyrazine derivative, a pyrazoline derivative, a pyrazolidine derivative, a piperidine derivative, a piperazine derivative, a morpholine derivative, an indole derivative, an isoindole derivative, a 1H-indazole derivative, an indoline derivative, a quinoline derivative (for example, quinoline, 3-quinolinecarbonitrile, or the like), an isoquinoline derivative, a cinnoline derivative, a quinazoline derivative, a quinoxaline derivative, a phthalazine derivative, a purine derivative, a pteridine derivative, a carbazole derivative, a phenanthridine derivative, an acridine derivative, a phenazine derivative, a 1,10-phenanthroline derivative, an adenine derivative, an adenosine derivative, a guanine derivative, a guanosine derivative, an uracil derivative, and an uridine derivative.

Examples of the above-mentioned non-nucleophilic counter ion of X⁻ include a halide ion such as a chloride ion and a bromide ion; fluoroalkyl sulfonate such as triflate, 1,1,1-trifluoroethanesulfonate, and nonafluorobutanesulfonate; arylsulfonate such as tosylate, benzenesulfonate, 4-fluorobenzenesulfonate, 1,2,3,4,5-pentafluorobenzenesulfonate; alkylsulfonate such as mesylate and butanesulfonate; imide acid such as bis(trifluoromethylsulfonyl) imide, bis(perfluoroethylsulfonyl)imide, and bis(perfluorobutylsulfonyl)imide; and methide acid such as tris(trifluoromethylsulfonyl)methide and tris(perfluoroethylsulfonyl)methide, and further include α-fluoro-substituted sulfonate shown by the following general formula (7) and α- and β- fluoro-substituted sulfonate shown by the following general formula (8).

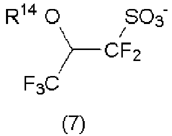

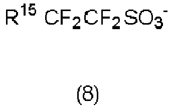

In the above-mentioned general formula (7), R¹⁴ represents a hydrogen atom, or a linear, branched, or cyclic alkyl group having 1 to 23 carbon atoms, an acyl group, an alkenyl group having 2 to 20 carbon atoms, an aryl group having 6 to 20 carbon atoms, or an aryloxy group. In the above-mentioned general formula (8), R¹⁵ represents a hydrogen atom, or a linear, branched, or cyclic alkyl group having 1 to 20 carbon atoms, an alkenyl group having 2 to 20 carbon atoms, or an aryl group having 6 to 20 carbon atoms.

Specific examples of the above-mentioned thermal acid generator can include the following, but the thermal acid generator is not limited thereto.

(CH₃CH₂),N⁺H

C₄F₉SO₃⁻

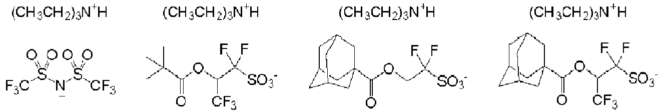

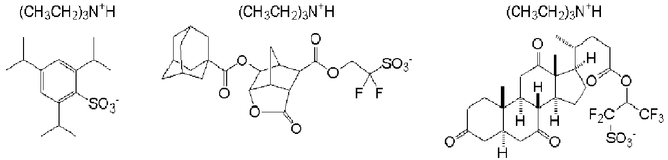

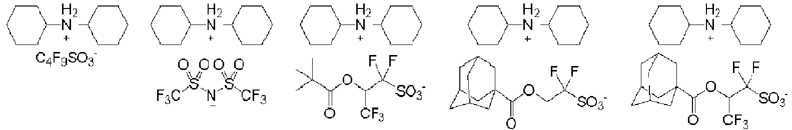

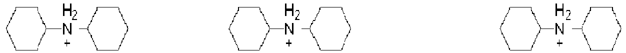

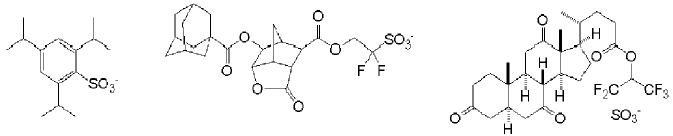

One kind of the thermal acid generator (B) contained in the adhesion film forming composition can be used alone, or two or more kinds thereof can be used in combination. The thermal acid generator is only required to be contained in the adhesion film forming composition, and an additive amount of the thermal acid generator is not particularly limited, and preferably 0.05 to 30 parts, more preferably 0.1 to 10 parts relative to 100 parts of the above-mentioned polymer compound (A). If the thermal acid generator is not contained, an amount of acid generation and cross-linking reaction become insufficient. When the additive amount is 0.05 parts or more, the amount of acid generation and the cross-linking reaction become sufficient. When the additive amount is 30 parts or less, there is a low possibility for occurrence of mixing phenomenon due to movement of acid toward the upper layer resist.

### (C) Organic Solvent

The adhesion film forming composition used in the present invention contains the organic solvent (C). The organic solvent is only required to be capable of dissolving the polymer compound (A) and the thermal acid generator (B) described above, and is not particularly limited. In a case where additives (a photo-acid generator (D), a cross-linker (E), a surface-active agent (F)), which will be described later, are added, the organic solvent is preferably capable of dissolving these additives. Specific examples include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol; ethers such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone, which are described in paragraphs [0144] to [0145] of JP 2008-111103 A. A mixture of one kind or two or more kinds thereof is preferably used.

The organic solvent is only required to be contained in the adhesion film forming composition, and an additive amount of the organic solvent is not particularly limited, and preferably 5,000 parts or more, more preferably 8,000 parts or more relative to 100 parts of the above-mentioned polymer compound (A).

The adhesion film forming composition used in the present invention can contain the photo-acid generator (D), the cross-linker (E), and the surface-active agent (F), which will be described below.

### (D) Photo-Acid Generator

The photo-acid generator (D) can be added to the adhesion film forming composition used in the present invention to adjust the pattern shape and exposure sensitivity of the resist upper layer film or the like appropriately. One kind of the photo-acid generator can be used alone, or two or more kinds thereof can be used in combination. As the photo-acid generator, for example, a photo-acid generator described in paragraphs [0160] to [0179] in JP 2009-126940 A can be used. An additive amount of the photo-acid generator is preferably 0.05 to 30 parts, more preferably 0.1 to 10 parts relative to 100 parts of the above-mentioned polymer compound (A). If the additive amount of the photo-acid generator is within the above-mentioned range, resolution is favorable and there is no possibility for occurrence of an issue of foreign substances after development of the resist or at the time of peeling.

### (E) Cross-Linker

In addition, the cross-linker (E) can also be added to the adhesion film forming composition used in the present invention to increase curability and further prevent inter-mixing with the resist upper layer film. The cross-linker is not particularly limited, and a wide range of publicly-known cross-linkers of various kinds of systems can be used. Examples of the cross-linker can include a melamine-based cross-linker, a glycoluril-based cross-linker, a benzoguanamine-based cross-linker, a urea-based cross-linker, a β-hydroxyalkylamide-based cross-linker, an isocyanurate-based cross-linker, an aziridine-based cross-linker, an oxazoline-based cross-linker, an epoxy-based cross-linker, and a phenol-based cross-linker. One kind of the above-mentioned cross-linker (E) can be used alone, or two or more kinds thereof can be used in combination. When the cross-linker is added, an additive amount of the cross-linker is preferably 5 to 50 parts, more preferably 10 to 40 parts relative to 100 parts of the above-mentioned polymer compound (A). When the additive amount is 5 parts or more, sufficient curability is manifested, and inter-mixing with the resist upper layer film can be prevented. In contrast, when the additive amount is 50 parts or less, there is no possibility for degradation of adhesiveness due to a decreased ratio of the polymer compound (A) in the adhesion film forming composition.

Specific examples of the melamine-based cross-linker can include hexamethoxymethylated melamine, hexabutoxymethylated melamine, alkoxy and/or hydroxy-substituted products thereof, and partially selfcondensation products thereof.

Specific examples of the glycoluril-based cross-linker can include tetramethoxymethylated glycoluril, tetrabutoxymethylated glycoluril, alkoxy and/or hydroxy-substituted products thereof, and partially selfcondensation products thereof.

Specific examples of the benzoguanamine-based cross-linker can include tetramethoxymethylated benzoguanamine, tetrabutoxymethylated benzoguanamine, alkoxy and/or hydroxy-substituted products thereof, and partially self- condensation products thereof.

Specific examples of the urea-based cross-linker include dimethoxymethylated dimethoxyethyleneurea, alkoxy and/or hydroxy-substituted products thereof, and partially self- condensation products thereof.

Specific examples of the β-hydroxyalkylamide-based cross-linker can include N,N,N',N'-tetra(2-hydroxyethyl)adipic acid amide.

Specific examples of the isocyanurate-based cross-linker can include triglycidyl isocyanurate and triallyl isocyanurate.

Specific examples of the aziridine-based cross-linker can include 4,4'-bis(ethyleneiminocarbonylamino)diphenylmethane and 2,2-bishydroxymethylbutanol-tris[3-(1-aziridinyl)propionate].

Specific examples of the oxazoline-based cross-linker can include 2,2'-isopropyridenebis(4-benzyl-2-oxazoline), 2,2'-isopropyridenebis(4-phenyl-2-oxazoline), 2,2'-methylenebis 4,5-diphenyl-2-oxazoline, 2,2'-methylenebis-4-phenyl-2-oxazoline, 2,2'-methylenebis-4-tertbutyl-2-oxazoline, 2,2'-bis(2-oxazoline), 1,3-phenylenebis(2-oxazoline), 1,4-phenylenebis(2-oxazoline), and a 2-isopropenyloxazoline copolymer.

Specific examples of the epoxy-based cross-linker can include diglycidyl ether, ethylene glycol diglycidyl ether, 1,4-butanediol diglycidyl ether, 1,4-cyclohexanedimethanol diglycidyl ether, poly(glycidyl methacrylate), trimethylol ethane triglycidyl ether, trimethylol propane triglycidyl ether, and pentaerythritol tetraglycidyl ether.

Specific examples of the phenol-based cross-linker can include a compound shown by the following general formula (9). 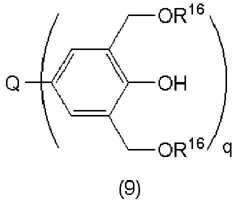 (In the formula, Q represents a single bond, or a q-valent hydrocarbon group having 1 to 20 carbon atoms. R¹⁶ is a hydrogen atom or an alkyl group having 1 to 20 carbon atoms. q is an integer of 1 to 5.)

Q represents a single bond, or a q-valent hydrocarbon group having 1 to 20 carbon atoms. q is an integer of 1 to 5, preferably 2 or 3. Examples of Q can include methane, ethane, propane, butane, isobutane, pentane, cyclopentane, hexane, cyclohexane, methylpentane, methylcyclohexane, dimethylcyclohexane, trimethylcyclohexane, benzene, toluene, xylene, ethylbenzene, ethyl isopropyl benzene, diisopropylbenzene, methylnaphthalene, ethylnaphthalene, and eicosane.

R¹⁶ is a hydrogen atom or an alkyl group having 1 to 20 carbon atoms. Specific examples of the alkyl group having 1 to 20 carbon atoms can include a methyl group, an ethyl group, a propyl group, an isopropyl group, a butyl group, an isobutyl group, a pentyl group, an isopentyl group, a hexyl group, an octyl group, an ethylhexyl group, a decyl group, and an eicosanyl group. R¹⁶ is preferably the hydrogen atom or the methyl group.

Specific examples of the compound shown by the above-mentioned general formula (9) can include the following compounds. Among these compounds, hexamethoxymethylated forms of triphenolmethane, triphenolethane, 1,1,1-tris(4-hydroxyphenyl)ethane, tris(4-hydroxyphenyl)-1-ethyl-4-isopropyl benzene are preferable in terms of curability of the adhesion film and improvement of film thickness uniformity.

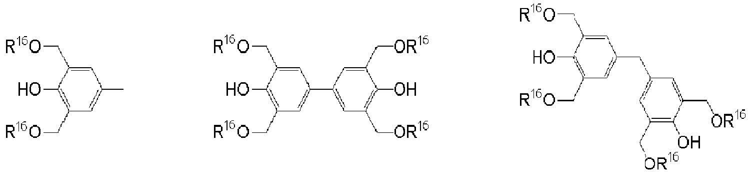

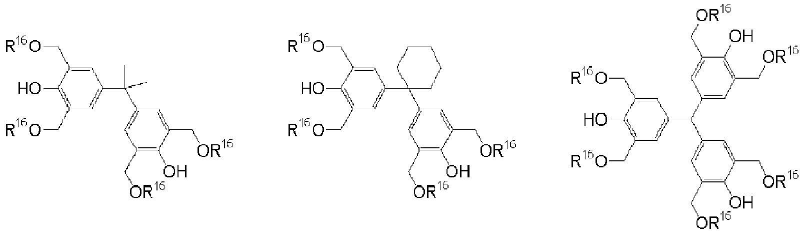

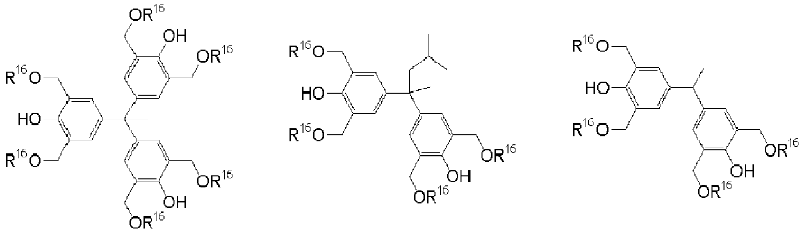

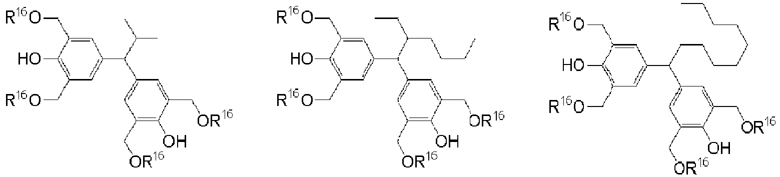

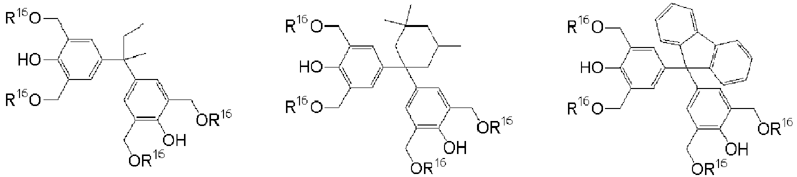

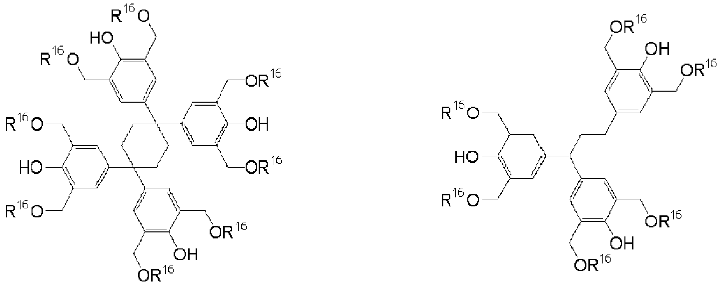

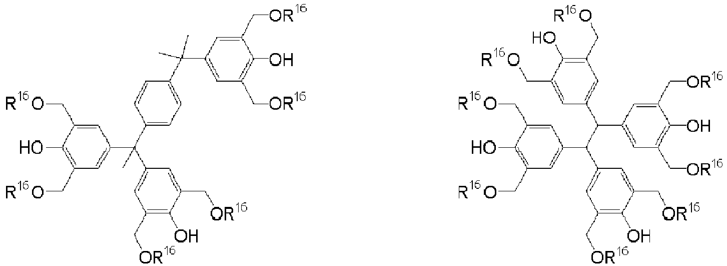

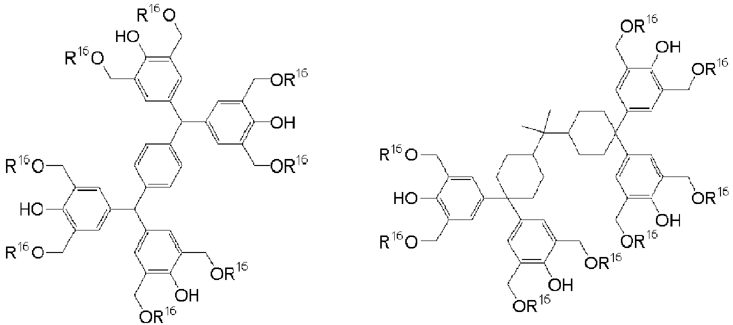

One kind of the above-mentioned cross-linker (E) can be used alone, or two or more kinds thereof can be used in combination. An additive amount of the cross-linker is preferably 10 to 50% by mass, more preferably 15 to 30% by mass relative to 100 parts of the above-mentioned polymer compound (A). When the additive amount is 10% by mass or more, sufficient curability is manifested, and inter-mixing with the resist upper layer film can be prevented. In contrast, when the additive amount is 50% by mass or less, there is no possibility for degradation of adhesiveness due to a decreased ratio of the polymer compound (A) in the adhesion film forming composition.

### (F) Surface-active Agent

The surface-active agent (F) can be added to the adhesion film forming composition used in the present invention to improve application properties in spin-coating. One kind of the surface-active agent can be used alone, or two or more kinds thereof can be used in combination. As the surface-active agent, for example, a surface-active agent described in paragraphs [0142] to [0147] in JP 2009-269953 A can be used. An additive amount of the surface-active agent is preferably 0.001 to 20 parts, more preferably 0.01 to 10 parts relative to 100 parts of the above-mentioned polymer compound (A). With the additive amount of the surface-active agent being within such a range, the application properties are infallibly improved, and a thin, uniform adhesion film can be formed.

A plasticizer can also be added to the adhesion film forming composition used in the present invention. The plasticizer is not particularly limited, and a wide range of publicly-known plasticizers of various kinds of systems can be used. Examples of the plasticizer can include: low-molecular compounds such as phthalic acid esters, adipic acid esters, phosphoric acid esters, trimellitic acid esters, and citric acid esters; and polymers such as a polyether-based polymer, a polyester-based polymer, and a polyacetal-based polymer described in JP 2013-253227 A. An additive amount of the plasticizer is preferably 5 to 500% by mass relative to 100 parts of the above-mentioned polymer compound (A). With the additive amount of the plasticizer being within such a range, the pattern is excellently embedded and has excellent uniformity.

In addition, fine patterning is implemented by use of the above-mentioned adhesion film forming composition in the multilayer resist process such as the two-layer resist process, and the four-layer resist process using the resist underlayer film and the silicon-containing middle layer film.

The above-mentioned silicon-containing middle layer film can be the silicon-containing resist middle layer film or the inorganic hard mask middle layer film depending on the pattern forming method, which will be described later. The above-mentioned inorganic hard mask middle layer film is preferably selected from a silicon oxide film, a silicon nitride film, and a silicon oxide nitride film.

### Pattern Forming Method

The present invention provides a pattern forming method for forming the adhesion film that has high adhesiveness with respect to the resist upper layer film and that exhibits the effect of preventing collapse of the fine pattern in the fine patterning process using the multilayer resist method in the manufacturing process for the semiconductor apparatus, using the above-mentioned adhesion film forming composition.

The adhesion film is formed by coating of the above-mentioned adhesion film forming composition onto the substrate to be processed by a spin-coating method or the like. After the spin-coating, the organic solvent is evaporated, and baking (thermal treatment) for promoting cross-linking reaction is performed to prevent inter-mixing with the resist upper layer film and the silicon-containing middle layer film. The baking is preferably performed at a temperature of 100°C or more and 300°C or less for a period of time within a range of 10 to 600 seconds, more preferably performed at a temperature of 200°C or more and 250°C or less for a period of time within a range of 10 to 300 seconds. In consideration of damage on the adhesion film and influence on deformation of a wafer, an upper limit of a heating temperature in a wafer process of lithography is preferably 300°C or less, more preferably 250°C or less.

That is, the present invention provides the pattern forming method for forming the adhesion film by rotary application of the adhesion film forming composition, and thermal treatment on the substrate to which the adhesion film forming composition is applied at the temperature of 100°C or more and 300°C or less during the period of time in the range of 10 to 600 seconds.

The adhesion film used in the present invention can also be formed by coating of the adhesion film forming composition onto the substrate to be processed by the above-mentioned spin-coating method or the like, and burning and curing of the adhesion film forming composition in an atmosphere with an oxygen concentration of 0.1% or more and 21% or less. The adhesion film forming composition is burned in such an oxygen atmosphere, whereby a sufficiently cured film can be obtained.

That is, the present invention provides the pattern forming method for forming the adhesion film by rotary application of the adhesion film forming composition, and thermal treatment on the substrate to which the adhesion film forming composition is applied in the atmosphere with the oxygen concentration of 0.1% or more and 21% or less.

The atmosphere during baking is not limited to the air, inert gas such as N₂, Ar, and He may be sealed. At this time, the atmosphere may be an atmosphere with an oxygen concentration of less than 0.1%. A baking temperature or the like may be similar to that described above. This can promote cross-linking reaction at the time of formation of the adhesion film without degradation of the substrate to be processed even in a case where the substrate to be processed contains a material that is unstable to heating under the oxygen atmosphere.

That is, the present invention provides the pattern forming method for forming the adhesion film by rotary application of the adhesion film forming composition, and thermal treatment on the substrate to which the adhesion film forming composition is applied in the atmosphere with the oxygen concentration of less than 0.1%.

The present invention provides a pattern forming method for forming a pattern on a substrate to be processed, the method comprising steps of:
(I-1) applying the adhesion film forming composition onto the substrate to be processed and thereafter performing thermal treatment to form an adhesion film;
(I-2) forming a resist upper layer film on the adhesion film using a resist upper layer film forming composition;
(I-3) performing pattern exposure on the resist upper layer film and thereafter developing the resist upper layer film with a developer to form a circuit pattern on the resist upper layer film; and
(I-4) transferring a pattern to the adhesion film and the substrate to be processed by dry etching with the resist upper layer film on which the circuit pattern is formed as a mask.

The pattern forming method according to the present invention is described below using an example of the four-layer resist process, but is not limited to this process. First, the present invention provides the pattern forming method for forming a pattern on a substrate to be processed, the method comprising at least steps of: forming a resist underlayer film on the substrate to be processed using an organic film forming composition; forming a silicon-containing middle layer film (silicon-containing resist middle layer) on the resist underlayer film using a resist middle layer film forming composition containing silicon atoms; forming an adhesion film on the silicon-containing resist middle layer film using the above-mentioned adhesion film forming composition; forming a resist upper layer film on the adhesion film using a resist upper layer film forming composition to form a multilayer resist film; exposing a pattern circuit area of the resist upper layer film and thereafter developing the resist upper layer film with a developer to form a resist upper layer film pattern; performing etching on the adhesion film and the silicon-containing resist middle layer film with the obtained resist upper layer film pattern as an etching mask to form a silicon-containing resist middle layer film pattern; performing etching on the resist underlayer film with the obtained silicon-containing resist middle layer film pattern as an etching mask to form a resist underlayer film pattern; and furthermore, performing etching on the substrate to be processed with the obtained resist underlayer film pattern as an etching mask to form a pattern on the substrate to be processed.

That is, the present invention provides a pattern forming method for forming a pattern on a substrate to be processed, the method comprising steps of:
(II-1) forming a resist underlayer film on the substrate to be processed;
(II-2) forming a silicon-containing resist middle layer film on the resist underlayer film;
(II-3) applying the adhesion film forming composition onto the silicon-containing resist middle layer film and thereafter performing thermal treatment to form an adhesion film;
(II-4) forming a resist upper layer film on the adhesion film using a resist upper layer film forming composition;
(II-5) performing pattern exposure on the resist upper layer film and thereafter developing the resist upper layer film with a developer to form a circuit pattern on the resist upper layer film;
(II-6) transferring a pattern to the adhesion film and the silicon-containing resist middle layer film by dry etching with the resist upper layer film on which the circuit pattern is formed as a mask;
(II-7) transferring a pattern to the resist underlayer film by dry etching with the silicon-containing resist middle layer film to which the pattern is transferred as a mask; and
(II-8) processing the substrate to be processed to form a pattern on the substrate to be processed with the resist underlayer film to which the pattern is transferred as a mask.

As the silicon-containing resist middle layer film in the above-mentioned four-layer resist process, a polysilsesquioxane-based middle layer film is also preferably used. The silicon-containing resist middle layer film has an antireflection effect, whereby reflection can be suppressed. Particularly, for 193 nm light exposure, if a material containing a large amount of aromatic groups and having high substrate etching resistance is used for the resist underlayer film, a k-value becomes high and substrate reflectance increases. However, with use of the silicon-containing resist middle layer film, the substrate reflectance can be made 0.5% or less. As the silicon-containing resist middle layer film having the antireflection effect, anthracene is preferably used for 248 nm or 157 nm light exposure, and polysilsesquioxane in which a phenyl group or a light-absorbing group having a silicon-silicon bond is suspended and that is cross-linked by acid or heat is preferably used for 193 nm light exposure.

In this case, formation of the silicon-containing resist middle layer film by the spin-coating method is simpler and more advantageous in terms of costs than formation by the CVD method.

Alternatively, the inorganic hard mask middle layer film may be formed as the silicon-containing middle layer film. In this case, a pattern can be formed on a substrate to be processed at least by forming a resist underlayer film on the substrate to be processed using an organic film forming composition; forming an inorganic hard mask middle layer film selected from a silicon oxide film, a silicon nitride film, and a silicon oxide nitride film on the resist underlayer film; forming an adhesion film on the inorganic hard mask middle layer film using the above-mentioned adhesion film forming composition; forming a resist upper layer film on the adhesion film using a photoresist upper layer film forming composition; exposing a pattern circuit area of the resist upper layer film and thereafter developing the resist upper layer film with a developer to form a resist upper layer film pattern on the resist upper layer film; performing etching on the adhesion film and the inorganic hard mask middle layer film with the obtained resist upper layer film pattern as an etching mask to form an inorganic hard mask middle layer film pattern; performing etching on the resist underlayer film with the obtained inorganic hard mask middle layer film pattern as an etching mask to form a resist underlayer film pattern; and furthermore, performing etching on the substrate to be processed with the obtained resist underlayer film pattern as an etching mask to form a pattern on the substrate to be processed.

That is, the present invention provides a pattern forming method for forming a pattern on a substrate to be processed, the method comprising steps of:
(III-1) forming a resist underlayer film on the substrate to be processed;
(III-2) forming, on the resist underlayer film, an inorganic hard mask middle layer film selected from the group consisting of a silicon oxide film, a silicon nitride film, and a silicon oxide nitride film;
(III-3) applying the adhesion film forming composition onto the inorganic hard mask middle layer film and thereafter performing thermal treatment to form an adhesion film;
(III-4) forming a resist upper layer film on the adhesion film using a resist upper layer film forming composition;
(III-5) performing pattern exposure on the resist upper layer film and thereafter developing the resist upper layer film with a developer to form a circuit pattern on the resist upper layer film;
(III-6) transferring a pattern to the adhesion film and the inorganic hard mask middle layer film by dry etching with the resist upper layer film on which the circuit pattern is formed as a mask;
(III-7) transferring a pattern to the resist underlayer film by dry etching with the inorganic hard mask middle layer film to which the pattern is transferred as a mask; and
(III-8) processing the substrate to be processed to form a pattern on the substrate to be processed with the resist underlayer film to which the pattern is transferred as a mask.

As described above, in the case of forming the inorganic hard mask middle layer film on the resist underlayer film, a silicon oxide film, a silicon nitride film, and a silicon oxide nitride film (SiON film) can be formed by the CVD method, the ALD method, or the like. Particularly, the inorganic hard mask intermediate film is preferably formed by the CVD method or the ALD method. A method of forming the silicon nitride film is described in, for example, JP 2002-334869 A and WO 2004/066377 A1. The film thickness of the inorganic hard mask middle layer film is preferably 5 to 200 nm, more preferably 10 to 100 nm. As the inorganic hard mask middle layer film, a SiON film that is highly effective as an antireflection film is the most preferably used. Since a substrate temperature becomes 300 to 500°C at the time of formation of the SiON film, the resist underlayer film needs to withstand the temperature of 300 to 500°C.

The adhesion film used in the present invention has a film thickness of 15 nm immediately below the resist upper layer film.

Under the circumstances where the resist upper layer film has become thinner along with the miniaturization of the pattern and there is no choice but to adopt design that decreases dry etching resistance from a perspective of resolution, the adhesion film immediately below the resist is required to be made as thin as possible in order to reduce load on the resist pattern in etching. Thus, it is preferable that the film thickness of the adhesion film used in the present invention be 15 nm or less, particularly 10 nm or less. The polymer compound that is contained in the resist underlayer film reported in Patent Document 8 is contained in the adhesion film forming composition used in the present invention. The polymer compound exhibits a higher etching rate than that of the resist upper layer film, and is preferable in etching.

When the adhesion film having the film thickness of 15 nm or less is formed from the adhesion film forming composition used in the present invention, spin-coating is preferably used. The adhesion film can be formed by setting a concentration of the above-mentioned polymer compound (A) contained in the adhesion film forming composition and/or the number of rotations as appropriate.

The resist upper layer film in the above-mentioned four-layer process may be of a positive-type or a negative-type. The resist upper layer film is preferably formed using a resist upper layer film forming composition containing at least a metallic atom-containing compound and an organic solvent. It is more preferable that the metallic atom-containing compound contain at least one selected from titanium, cobalt, copper, zinc, zirconium, lead, indium, tin, antimony, and hafnium. After the resist upper layer film forming composition is spin-coated, pre-baking is performed preferably at a temperature of 60 to 180°C for a period of time in a range of 10 to 300 seconds. Thereafter, according to a routine method, exposure is performed, furthermore, post-exposure baking (PEB) is performed, and development is performed, whereby the resist upper layer film pattern is obtained. Note that the thickness of the resist upper layer film is not particularly limited, but preferably 20 to 300 nm, and more preferably 25 to 250 nm.

A circuit pattern (resist upper layer film pattern) is formed on the resist upper layer film. In formation of the circuit pattern, the circuit pattern is preferably formed by photolithography with a wavelength of 10 nm or more and 300 nm or less, electron beam direct drawing, nanoimprinting, or a combination thereof.

Examples of exposure light can include high-energy rays with a wavelength of 300 nm or less, specifically, far-ultraviolet rays, KrF excimer laser light (248 nm) and ArF excimer laser light (193 nm), F₂ laser light (157 nm), Kr₂ laser light (146 nm), Ar₂ laser light (126 nm), soft X-rays (EUV) having a wavelength of 3 to 20 nm, electron beams (EB), ion beams, X-rays, and the like.

In formation of the circuit pattern, as a development method, alkaline development or development with an organic solvent is preferably used to develop the circuit pattern.

Subsequently, etching is performed with the obtained resist upper layer film pattern as a mask. The adhesion film used in the present invention exhibits a higher etching rate, and can be used as a thin film with a thickness of 15 nm or less, whereby it is possible to reduce load on etching on the resist pattern serving as the mask. Thus, it is possible to perform etching on the adhesion film, the film immediately below the adhesion film, for example, the silicon-containing resist middle layer film and the inorganic hard mask middle layer film. Etching is performed on the silicon-containing resist middle layer film and the inorganic hard mask middle layer film using fluorocarbon gas. With this process, the silicon-containing resist middle layer film pattern or the inorganic hard mask middle layer film pattern is formed.

Subsequently, etching is performed on the resist underlayer film with the obtained silicon resist upper layer film pattern or as the inorganic hard mask middle layer film pattern a mask.

The subsequent etching on the substrate to be processed can also be performed according to the routine method. For example, when the substrate to be processed is a SiO₂-, SiN-, or silica-based low-dielectric insulation film, etching mainly using fluorocarbon gas is performed. When the substrate to be processed is made of p-Si, Al, or W, etching mainly using chlorine-or bromine-based gas is performed. In a case where etching is performed on the substrate to processed with fluorocarbon gas, the silicon-containing middle layer film pattern is removed simultaneously at the time of processing on the substrate. In a case where etching is performed on the substrate with chlorine-or bromine-based gas, peeling of the silicon-containing middle layer film needs to be performed separately as dry etching peeling with fluorocarbon gas after the substrate is processed.

Note that the substrate to be processed is not particularly limited, and a semiconductor apparatus substrate, or the semiconductor apparatus substrate on which any one of a metal film, a metal carbide film, a metal oxide film, a metal nitride film, a metal oxide carbide film, and a metal oxide nitride film is formed can be used. As the metal, silicon, titanium, tungsten, hafnium, zirconium, chrome, germanium, copper, silver, gold, aluminum, indium, gallium, arsenic, palladium, iron, tantalum, iridium, cobalt, manganese, molybdenum, or an alloy thereof can be used.

Specifically, the substrate made of Si, α-Si, p-Si, SiO₂, SiN, SiON, W, TiN, Al, or the like, the substrate on which a layer to be processed is formed, or the like is used. As the layer to be processed, a low-k film made of Si, SiO₂, SiON, SiN, p-Si, α-Si, W, W-Si, Al, Cu, Al-Si, or the like and a stopper film for the low-k film are used, and the layer to be processed can be formed to have generally a thickness of 50 to 10,000 nm, participially a thickness of 100 to 5,000 nm. Note that in a case where the layer to be processed is formed, the substrate and the layer to be processed having different materials are used.

Specific examples of the four-layer resist process are as described below with reference to FIG. 1. In the case of the four-layer resist process, as shown in FIG. 1(A), a resist underlayer film 3 is formed on a layer to be processed 2 laminated on a substrate 1 using an organic material, thereafter a silicon-containing middle layer film 4 is formed, an adhesion film 5 is formed on the silicon-containing middle layer film 4 using the adhesion film forming composition according to the present invention, and a resist upper layer film 6 is formed on the adhesion film 5.

Subsequently, as shown in FIG. 1(B), an exposed part 7 of the resist upper layer film is exposed, PEB is performed, and development is performed to form a resist upper layer film pattern 6a (FIG. 1(C)). Etching is performed also on the adhesion film 5 with the obtained resist upper layer film pattern 6a as a mask to form an adhesion film pattern 5a (FIG. 1(D)). Furthermore, etching is performed also on the silicon-containing middle layer film 4 using CF-based gas to form a silicon-containing middle layer film pattern 4a (FIG. 1(E)). Etching is performed on the resist underlayer film 3 using O₂-based gas with the obtained silicon-containing middle layer film pattern 4a as a mask to form a resist underlayer film pattern 3a (FIG. 1(F)). Furthermore, the silicon-containing middle layer film pattern 4a is removed, and thereafter etching is performed on the layer to be processed 2 with the resist underlayer film pattern 3a as a mask to form a pattern 2a (FIG. 1(G)).

In this manner, the pattern forming method according to the present invention allows for formation of the fine pattern on the substrate to be processed in the multilayer resist process.

### EXAMPLE

Hereinafter, the present invention will be specifically described using Synthesis Examples, Examples, and Comparative Examples. However, the present invention is not limited to the following Examples. Note that a molecular weight was measured by gel permeation chromatography (GPC) using tetrahydrofuran (THF) as an eluant, and a degree of dispersion (Mw/Mn) was obtained from a weight-average molecular weight (Mw) and a number average molecular weight (Mn) in terms of polystyrene.

### Synthesis Example 1: Synthesis of Polymer Compound (A1)

Propylene glycol monomethyl ether acetate (hereinafter referred to as "PGMEA") in an amount of 38.9 g was heated and stirred at 80°C in a nitrogen atmosphere. A mixture of glycidyl methacrylate in an amount of 29.7 g, tert-butyl acrylate in an amount of 8.9 g, benzyl acrylate in an amount of 11.3 g, and PGMEA in an amount of 38.9 g, and a mixture of dimethyl 2,2-azobis (2-methylpropionate) in an amount of 4.0 g and PGMEA in an amount of 38.9 g were added thereto simultaneously and separately for four hours. The above was further heated and stirred for 20 hours, and cooled to a room temperature, whereby a PGMEA solution of an intended polymer compound (A1) was obtained. As a result of analysis, a weight-average molecular weight (Mw) of the polymer compound (A1) was 12,000, and a degree of dispersion (Mw/Mn) was 2.4.

### Synthesis Example 2: Synthesis of Polymer Compound (A2)

PGMEA in an amount of 38.9 g was heated and stirred at 80°C in a nitrogen atmosphere. A mixture of glycidyl methacrylate in an amount of 29.7 g, tert-butyl acrylate in an amount of 8.9 g, benzyl acrylate in an amount of 11.3 g, and PGMEA in an amount of 38.9 g, and a mixture of dimethyl 2,2-azobis (2-methylpropionate) in an amount of 4.0 g and PGMEA in an amount of 38.9 g were added thereto simultaneously and separately for four hours. The above was further heated and stirred for 20 hours and cooled to 60°C. After heptanone in an amount of 200 g was added thereto, the above was cooled to a room temperature, and left for two hours. An upper layer was separated and removed, PGMEA in an amount of 100 g was added thereto, heptanone was distilled off under reduced pressure, whereby a PGMEA solution of an intended polymer compound (A2) was obtained. As a result of analysis, a weight-average molecular weight (Mw) of the polymer compound (A2) was 13,000, and a degree of dispersion (Mw/Mn) was 2.2.

### Synthesis Examples 3 to 7: Synthesis of Polymer Compounds (A3) to (A7)

PGMEA solutions of polymer compounds (A3) to (A7) were obtained by a method similar to that used in Synthesis Example 1 except a kind of a raw material monomer and a mole ratio were changed in accordance with a structure of each polymer compound. Weight-average molecular weights (Mw) and degrees of dispersion (Mw/Mn) of the polymer compounds (A3) to (A7) were as follows.

The polymer compounds (A1) to (A7) synthesized in Synthesis Examples are shown below.

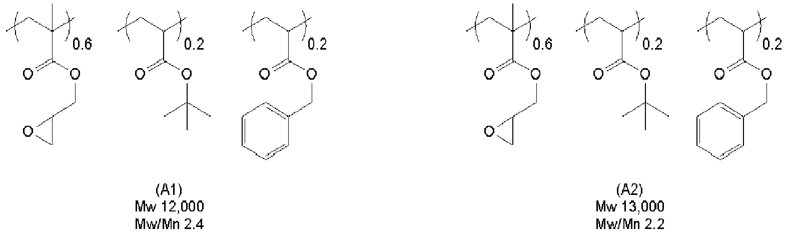

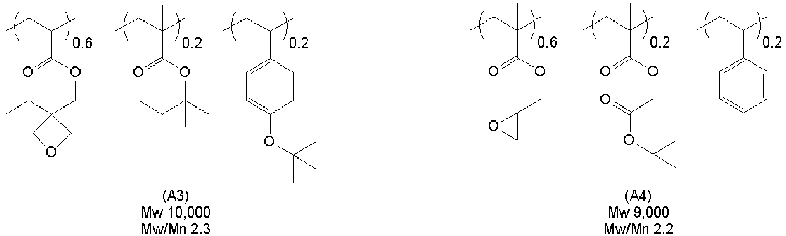

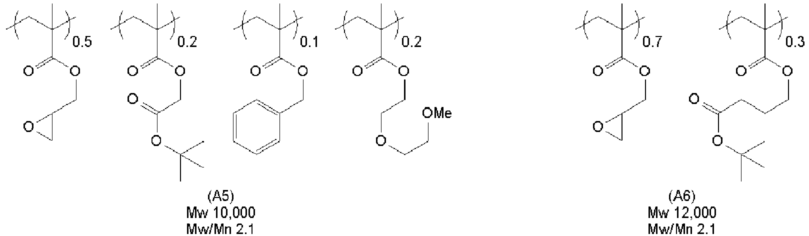

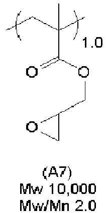

### Preparation of Adhesion film Forming Composition (AL1 to 16, Comparison AL1)

For preparation of the adhesion film forming composition, the above-mentioned polymer compounds (A1) to (A7), (AG1) to (AG3) as thermal acid generators, (AG4) to (AG5) as photo-acid generators, and (X1) to (X2) as cross-linkers were used. These components were dissolved in an organic solvent containing PF636 (from OMNOVA) in an amount of 0.001% by mass in ratios shown in Table 1, and thereafter the solvent was filtrated with a 0.1 um fluororesin filter, whereby the adhesion film forming compositions (AL1 to 16, Comparison AL1) were prepared.

(CH₃CH₂)₃N⁺H C₄F₉SO₃⁻ (AG1)

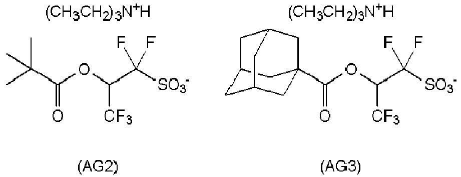

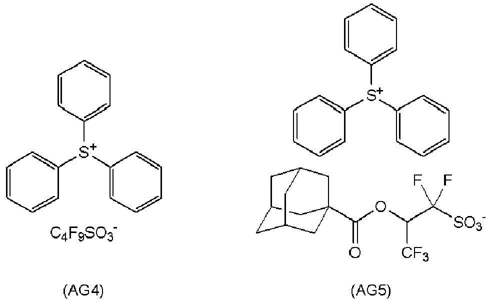

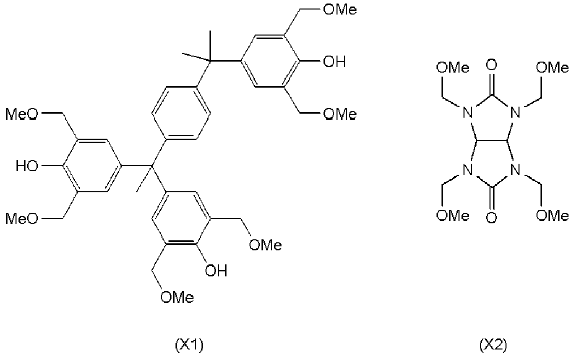

**[Table 1]**

| Adhesion film forming composition | Polymer compound (parts by mass) | Thermal acid generator (parts by mass) | Photo-acid generator (parts by mass) | Crosslinker (parts by mass) | Organic solvent (parts by mass) |
|---|---|---|---|---|---|
| AL1 | A1 (10) | AG1 (0.1) | | | PGMEA (2,240) PGME (960) |
| AL2 | A1 (10) | AG1 (0.1) | AG4 (0.2) | | PGMEA (2,240) PGME (960) |
| AL3 | A1 (10) | AG1 (0.1) | AG5 (0.2) | X1 (0.2) | PGMEA (2,240) PGME (960) |
| AL4 | A2 (10) | AG1 (0.1) | | | PGMEA (2,240) PGME (960) |
| AL5 | A2 (10) | AG2 (0.1) | AG4 (0.4) | | PGMEA (2,240) PGME (960) |
| AL6 | A3 (10) | AG1 (0.1) | | | PGMEA (3,200) |
| AL7 | A4 (10) | AG1 (0.1) | | | PGMEA (3,200) |
| AL8 | A4 (10) | AG1 (0.1) | | X2 (0.2) | PGMEA (3,200) |
| AL9 | A5 (10) | AG1 (0.1) | | | PGMEA (3,200) |
| AL10 | A5 (10) | AG2 (0.1) | AG4 (0.4) | | PGMEA (3,200) |
| AL11 | A5 (10) | AG3 (0.1) | | | PGMEA (3,200) |
| AL12 | A5 (10) | AG3 (0.1) | | | PGMEA (1,400) |
| AL13 | A6 (10) | AG1 (0.1) | | | PGMEA (3,200) |
| AL14 | A6 (10) | AG2 (0.1) | AG4 (0.4) | | PGMEA (3,200) |
| AL15 | A6 (10) | AG3 (0.1) | | | PGMEA (3,200) |
| AL16 | A6 (10) | AG3 (0.1) | | | PGMEA (1,400) |
| Comparison AL1 | A7 (10) | AG1 (0.2) | AG4 (0.4) | | PGMEA (3,200) |

| | | | | | |
|---|---|---|---|---|---|
| PGMEA: propylene glycol monomethyl ether acetate PGME: propylene glycol monomethyl ether | | | | | |

### Example 1: Evaluation of Film Thickness Uniformity and Solvent Tolerance (Examples 1-1 to 1-16, Comparative Example 1-1)

Each of the adhesion film forming compositions prepared as described above (AL1 to 16 and Comparison AL1) was applied onto a 300 nm silicon substrate, and baked at 220°C for 60 seconds, thereafter a film thickness was measured. The film thickness was measured at 225 locations over the entire plane of a wafer, and a difference between maximum and minimum values of measured values was obtained as a parameter indicating film thickness uniformity. A smaller value means higher film thickness uniformity, and thus is favorable. A film thickness average value of values at these 225 locations was assumed to be a film thickness.

Solvent tolerance was evaluated by dispensing a PGMEA solvent over the obtained adhesion film; leaving the adhesion film for 30 seconds, thereafter performing spin-drying, and performing baking at 100°C for 60 seconds to evaporate the PGMEA solvent; measuring a film thickness again to obtain a difference in film thickness before and after PGMEA treatment. Results are shown in Table 2.

**[Table 2]**

| Examples | Adhesion film forming composition | Film thickness after film formation: a (Å) | Film thickness uniformity (Å) | Film thickness after solvent treatment: b (Å) | b/a x100 (%) |
|---|---|---|---|---|---|
| Ex. 1-1 | AL1 | 50.0 | 1.5 | 49.8 | 99.6 |
| Ex. 1-2 | AL2 | 50.3 | 1.4 | 50.1 | 99.6 |
| Ex. 1-3 | AL3 | 50.2 | 1.3 | 50.0 | 99.6 |
| Ex. 1-4 | AL4 | 50.5 | 1.2 | 50.3 | 99.6 |
| Ex. 1-5 | AL5 | 50.3 | 1.1 | 50.0 | 99.4 |
| Ex. 1-6 | AL6 | 50.4 | 1.6 | 50.2 | 99.6 |
| Ex. 1-7 | AL7 | 50.1 | 1.7 | 49.9 | 99.6 |
| Ex. 1-8 | AL8 | 50.4 | 1.3 | 50.1 | 99.4 |
| Ex. 1-9 | AL 9 | 50.0 | 1.5 | 49. 9 | 99.8 |
| Ex. 1-10 | AL10 | 50.2 | 1.4 | 50.0 | 99.6 |
| Ex. 1-11 | AL11 | 50.1 | 1.5 | 50.0 | 99.8 |
| Ex. 1-12 | AL12 | 150.1 | 1.5 | 149.9 | 99.9 |
| Ex. 1-13 | AL13 | 50.2 | 1.4 | 50.1 | 99.8 |
| Ex. 1-14 | AL14 | 50.4 | 1.3 | 50.3 | 99.8 |
| Ex. 1-15 | AL15 | 50.5 | 1.4 | 50.4 | 99.8 |
| Ex. 1-16 | AL16 | 150.3 | 1.5 | 150.0 | 99.8 |
| Comp. Ex. 1-1 | Comparison AL1 | 50.1 | 1.3 | 50.0 | 99.8 |

As shown in Table2, it is found that the film thickness in any of Examples 1-1 to 1-16 using the adhesion film forming compositions AL1 to 16 and Comparative Example 1-1 using Comparison AL1 for comparison was hardly decreased by the solvent treatment and the adhesion film has favorable solvent tolerance.

### Example 2: EUV Exposure Pattern Formation Test Using Positive Resist (Examples 2-1 to 2-16, Comparative Example 2-1)

Spin-on carbon ODL-301 (containing carbon in an amount of 88% by mass) from Shin-Etsu Chemical Co., Ltd. was applied onto a silicon wafer substrate and baking was performed at 350°C for 60 seconds to form a resist underlayer film having a film thickness of 100 nm. A silicon-containing spin-on hardmask SHB-A940 (containing silicon in an amount of 43% by mass) from Shin-Etsu Chemical Co. Ltd. was applied onto the resist underlayer film and baking was performed at 220°C for 60 seconds to form a silicon-containing middle layer film having a film thickness of 15 nm. Furthermore, the above-mentioned adhesion film forming composition (AL1 to 16, Comparison AL1) was applied thereto, and baking was performed at 220°C for 60 seconds to form an adhesion film having a film thickness of 5 nm (AL1 to 11, 13 to 15, Comparison AL1) or 15 nm (AL12, 16). A positive resist upper layer film forming composition described in Table 3 was applied onto the adhesion film, and baking was performed at 100°C for 60 seconds to form a resist upper layer film having a film thickness of 40 nm.

The positive resist upper layer film forming composition (monolayer resist for EUV) was prepared by dissolving a polymer compound PRP1 and a quencher Q1 in a solvent containing FC-4430 (from Sumitomo 3M Limited) in an amount of 0.1 mass% in a ratio shown in Table 3, and performing filtration with a 0.1 um fluororesin filter.

**[Table 3]**

| | Polymer compound (parts by mass) | Quencher (parts by mass) | Organic solvent (parts by mass) |
|---|---|---|---|
| Monolayer resist for EUV | PRP1 (100) | Q1 (0.25) | PGMEA/DAA (3,000/1,000) |

Polymer for resist: PRP1
Molecular weight Mw = 9,200
Dispersion Mw/Mn = 1.8

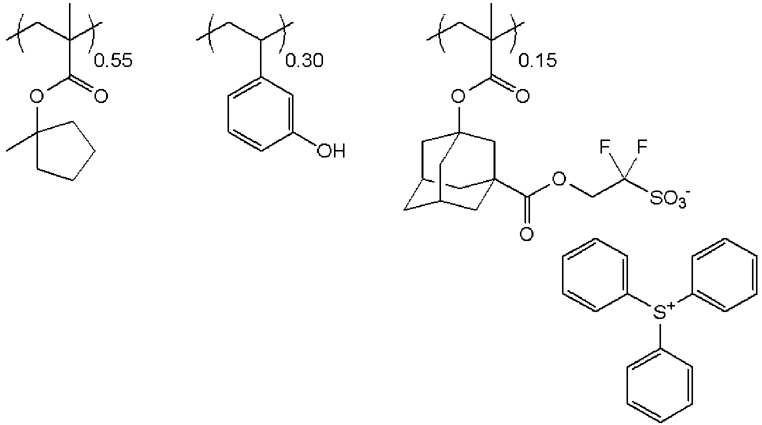

### Quencher: Q1

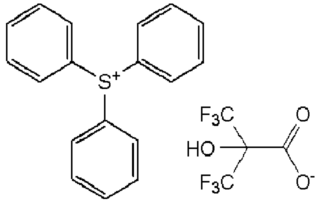

Subsequently, a 18 nm line-and-space (LS) 1: 1 pattern was exposed using an EUV exposure apparatus (ASML's EUV scanner NXE3400, NA0.33, σ0.9, 90 degree dipole illumination), and baking (PEB) was performed at 90°C for 60 seconds, development was performed with a 2.38% by mass tetramethylammonium hydroxide (TMAH) aqueous solution for 30 seconds, whereby a line-and-space pattern with a space width of 18 nm and a pitch of 36 nm was obtained. A cross-section shape and roughness were observed on this pattern using an electron microscope. By increasing an exposure amount in which the above-mentioned line-and-space pattern was formed little by little, a minimum size of a line that is resolved without being collapsed when a line size was thinned was obtained to serve as a collapse limit (nm). A smaller value exhibits higher collapse resistance, and thus is preferable.

The obtained pattern cross-section shape was evaluated with an electron microscope from Hitachi, Ltd. (S-4700), and pattern roughness was evaluated with an electron microscope from Hitachi High-Technologies Corporation (GC6300). Results are shown in Table 4.

**[Table 4]**

| Examples | Adhesion film forming composition | Pattern after development cross-section shape | Pattern roughness (nm) | Collapse limit (nm) |
|---|---|---|---|---|
| Ex. 2-1 | AL1 | Rectangular shape | 1.6 | 16 |
| Ex. 2-2 | AL2 | Rectangular shape | 1.7 | 17 |
| Ex. 2-3 | AL3 | Rectangular shape | 1.6 | 16 |
| Ex. 2-4 | AL4 | Rectangular shape | 1.5 | 16 |
| Ex. 2-5 | AL5 | Rectangular shape | 1.6 | 15 |
| Ex. 2-6 | AL6 | Rectangular shape | 1.8 | 16 |
| Ex. 2-7 | AL7 | Rectangular shape | 1.6 | 16 |
| Ex. 2-8 | AL8 | Rectangular shape | 1.5 | 16 |
| Ex. 2-9 | AL9 | Rectangular shape | 1.7 | 15 |
| Ex. 2-10 | AL10 | Rectangular shape | 1.6 | 16 |
| Ex. 2-11 | AL11 | Rectangular shape | 1.5 | 14 |
| Ex. 2-12 | AL12 | Rectangular shape | 1.5 | 15 |
| Ex. 2-13 | AL13 | Rectangular shape | 1.5 | 16 |
| Ex. 2-14 | AL14 | Rectangular shape | 1.6 | 15 |
| Ex. 2-15 | AL15 | Rectangular shape | 1.6 | 14 |
| Ex. 2-16 | AL16 | Rectangular shape | 1.7 | 15 |
| Comp. Ex. 2-1 | Comparison AL1 | Tapered shape | 2.5 | 18 |

As shown in Table 4, in Examples 2-1 to 2-16 using the adhesion film forming compositions AL1 to 16, a pattern has a rectangular shape, and exhibits excellent performance in preventing collapse. In contrast, in Comparative Example 2-1 using Comparison AL1, a pattern has a tapered shape and roughness is large. Combined use of the repeating units shown by the above-mentioned general formulae (1) and (2) in the polymer compound constituting the adhesion film is found to be effective.

### Example 3: Evaluation of Etching (Examples 3-1 to 3-16, Comparative Example 3-1)

Subsequent to the formation of the resist upper layer film pattern in Example 2, dry etching (pattern transfer) was performed on the silicon-containing middle layer film using an etching apparatus Telius from Tokyo Electron Limited with the resist upper layer film pattern as a mask, and dry etching (pattern transfer) was performed on the resist underlayer film with the obtained silicon-containing middle layer film pattern as a mask to form a resist underlayer film pattern. Etching conditions are as follows.

### (Transfer conditions of resist upper layer film pattern to silicon-containing middle layer film)

Chamber pressure: 10.0 Pa
RF power: 1500W
CF₄ gas flow rate: 75 mL/min
O₂ gas flow rate: 15 mL/min
Time: 15 sec

### (Transfer conditions of silicon-containing middle layer film pattern to resist underlayer film)

Chamber pressure: 2.0 Pa
RF power: 500W
Ar gas flow rate: 75 mL/min
O₂ gas flow rate: 45 mL/min
Time: 90 sec

Whether or not the resist underlayer film could be formed was checked by a sky-SEM observation (top-down SEM view) of a wafer after transfer (dry etching) of the pattern to the resist underlayer film. Evaluation results are shown in Table 5.

**[Table 5]**

| Examples | Adhesion film forming composition | Resist underlayer film pattern |
|---|---|---|
| Ex. 3-1 | AL1 | Can be formed |
| Ex. 3-2 | AL2 | Can be formed |
| Ex. 3-3 | AL3 | Can be formed |
| Ex. 3-4 | AL4 | Can be formed |
| Ex. 3-5 | AL5 | Can be formed |
| Ex. 3-6 | AL6 | Can be formed |
| Ex. 3-7 | AL7 | Can be formed |
| Ex. 3-8 | AL8 | Can be formed |
| Ex. 3-9 | AL9 | Can be formed |
| Ex. 3-10 | AL10 | Can be formed |
| Ex. 3-11 | AL11 | Can be formed |
| Ex. 3-12 | AL12 | Can be formed |
| Ex. 3-13 | AL13 | Can be formed |
| Ex. 3-14 | AL14 | Can be formed |
| Ex. 3-15 | AL15 | Can be formed |
| Ex. 3-16 | AL16 | Can be formed |
| Comp. Ex.3-1 | Comparison AL1 | Cannot be formed |

As shown in Table 5, in the pattern forming method according to the present invention (Examples 3-1 to 3-16), the resist upper layer film pattern was favorably transferred to the resist underlayer film both in the case where the film thickness of the adhesion film was 5 nm (Examples 3-1 to 3-11, 3-13 to 3-15) and in the case where the film thickness of the adhesion film was 15 nm (Examples 3-12, 3-16), and it is found that the adhesion films of AL1 to 16 are effective in fine patterning according to the multilayer resist method.

In contrast, in Comparative Example 3-1, the resist underlayer film could not be formed. This result attributes to a defective shape of the resist pattern.

### Example 4: Electron Beam Pattern Formation Test (Examples 4-1 to 4-16, Comparative Example 4-1)

Spin-on carbon ODL-301 (containing carbon in an amount of 88% by mass) from Shin-Etsu Chemical Co., Ltd. was applied onto a silicon wafer substrate and baking was performed at 350°C for 60 seconds to form a resist underlayer film having a film thickness of 100 nm. A silicon-containing spin-on hardmask SHB-A940 (containing silicon in an amount of 43% by mass) from Shin-Etsu Chemical Co. Ltd. was applied onto the resist underlayer film and baking was performed at 220°C for 60 seconds to form a silicon-containing middle layer film having a film thickness of 20 nm. Furthermore, each of the adhesion film forming compositions AL1 to 16 and Comparison AL1 was applied, baking was performed at 220°C for 60 seconds to form an adhesion film with a film thickness of 5 nm, a metal-containing resist upper layer film forming composition was applied onto the adhesion film, and baking was performed at 180°C for 60 seconds to form a resist upper layer film having a film thickness of 60 nm.

The metal-containing resist upper layer film was prepared by dissolving a titanium-containing compound MPRP1 and a metal salt sensitizer S1 in a 4-methyl-2-pentanol (MIBC) containing FC-4430 (from Sumitomo 3M Limited) in an amount of 0.1% by mass in a ratio shown in Table 6, and performing filtration with a 0.1 um fluororesin filter.

**[Table 6]**

| | titanium-containing compound (parts by mass) | metal salt sensitizer (parts by mass) | Organic solvent (parts by mass) |
|---|---|---|---|
| Metal-containing resist upper layer film forming composition | MPRP1 (100) | S1 (1) | MIBC (2,000) |

### Synthesis of Titanium-Containing Compound MPRP1

While titanium tetraisopropoxide (Tokyo Chemical Industry Co., Ltd.) in an amount of 284 g was stirred in a solution of 2-propanol (IPA) in an amount of 500 g, a solution of IPA in an amount of 500 g containing deionized water in an amount of 27 g was delivered by drops at a room temperature for two hours. 2,4-dimethyl-2,4-octanediol in an amount of 180 g was added to the obtained solution, and the solution was stirred for 30 minutes at a room temperature. The solution was condensed under reduced pressure at 30°C, thereafter further heated to 60°C, and kept heated under reduced pressure until a distillate was not produced. When the distillate no longer appeared, 4-methyl-2-pentanol (MIBC) in an amount of 1,200 g was added, and the solution was heated until IPA did not distillate under reduced pressure, whereby an MIBC solution of the titanium-containing compound MPRP1 in an amount of 1,000 g (the concentration of the compound being 25% by mass) was obtained. A molecular weight of the solution in terms of polystyrene was measured and Mw = 1,200 was obtained.

### Metal salt sensitizer: S1

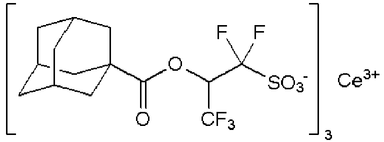

Subsequently, drawing was performed in a vacuum chamber at an acceleration voltage of 125 k using ELS-F125 (from ELIONIX INC.). Immediately after the drawing, baking (PEB) was performed at 200°C for 60 seconds, and paddle development with butyl acetate was performed for 20 seconds to obtain a negative pattern.

The obtained resist pattern was evaluated as follows. With an exposure amount in which a 50 nm line-and-space 1:1 pattern was exposed as sensitivity, a minimum size of a line that was resolved without being collapsed when a line size was thinned was obtained to serve as a collapse limit (nm). A smaller value exhibits higher collapse resistance, and thus is preferable. Results are shown in Table 7.

**[Table 7]**

| Examples | Adhesion film forming composition | Collapse limit (nm) |
|---|---|---|
| Ex. 4-1 | AL1 | 45 |
| Ex. 4-2 | AL2 | 45 |
| Ex. 4-3 | AL3 | 46 |
| Ex. 4-4 | AL4 | 46 |
| Ex. 4-5 | AL5 | 45 |
| Ex. 4-6 | AL6 | 45 |
| Ex. 4-7 | AL7 | 45 |
| Ex. 4-8 | AL8 | 45 |
| Ex. 4-9 | AL9 | 44 |
| Ex. 4-10 | AL10 | 45 |
| Ex. 4-11 | AL11 | 43 |
| Ex. 4-12 | AL12 | 44 |
| Ex. 4-13 | AL13 | 44 |
| Ex. 4-14 | AL14 | 44 |
| Ex. 4-15 | AL15 | 43 |
| Ex. 4-16 | AL16 | 43 |
| Comp. Ex.4-1 | Comparison AL1 | 50 |

As shown in Table 7, in Examples 4-1 to 4-16 using the adhesion film forming compositions AL1 to 16 have smaller values of the collapse limit than that in Comparative Example 4-1 using Comparison AL1. It is found that the present invention exhibits excellent adhesiveness also in the metal-containing resist, and is effective in fine patterning.

Consequently, the adhesion film obtained from the adhesion film forming composition using the polymer compound containing the specific repeating units provides the effect of preventing collapse of the fine resist pattern, and thus is extremely useful as the adhesion film used in the multilayer resist method. In addition, the pattern forming method using this adhesion film allows for transfer of the fine pattern to the substrate to be processed with high accuracy.

The present description includes the following embodiments.
[1]: A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
   (I-1) applying the adhesion film forming composition onto the substrate to be processed and thereafter performing thermal treatment to form an adhesion film;
   (I-2) forming a resist upper layer film on the adhesion film using a resist upper layer film forming composition;
   (I-3) performing pattern exposure on the resist upper layer film and thereafter developing the resist upper layer film with a developer to form a circuit pattern on the resist upper layer film; and
   (I-4) transferring a pattern to the adhesion film and the substrate to be processed by dry etching with the resist upper layer film on which the circuit pattern is formed as a mask, 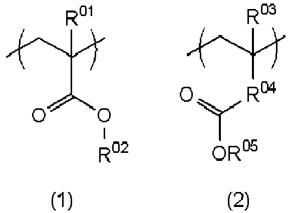 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 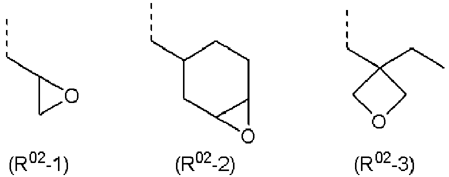 wherein a broken line represents a bond.
[2]: A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
   (II-1) forming a resist underlayer film on the substrate to be processed;
   (II-2) forming a silicon-containing resist middle layer film on the resist underlayer film;
   (II-3) applying the adhesion film forming composition onto the silicon-containing resist middle layer film and thereafter performing thermal treatment to form an adhesion film;
   (II-4) forming a resist upper layer film on the adhesion film using a resist upper layer film forming composition;
   (II-5) performing pattern exposure on the resist upper layer film and thereafter developing the resist upper layer film with a developer to form a circuit pattern on the resist upper layer film;
   (II-6) transferring a pattern to the adhesion film and the silicon-containing resist middle layer film by dry etching with the resist upper layer film on which the circuit pattern is formed as a mask;
   (II-7) transferring a pattern to the resist underlayer film by dry etching with the silicon-containing resist middle layer film to which the pattern is transferred as a mask; and
   (II-8) processing the substrate to be processed to form a pattern on the substrate to be processed with the resist underlayer film to which the pattern is transferred as a mask, 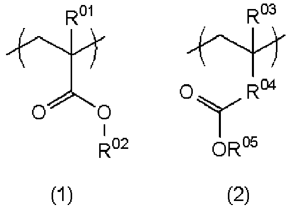 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 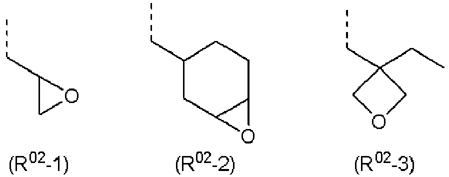 wherein a broken line represents a bond.
[3]: A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
   (III-1) forming a resist underlayer film on the substrate to be processed;
   (III-2) forming, on the resist underlayer film, an inorganic hard mask middle layer film selected from the group consisting of a silicon oxide film, a silicon nitride film, and a silicon oxide nitride film;
   (III-3) applying the adhesion film forming composition onto the inorganic hard mask middle layer film and thereafter performing thermal treatment to form an adhesion film;
   (III-4) forming a resist upper layer film on the adhesion film using a resist upper layer film forming composition;
   (III-5) performing pattern exposure on the resist upper layer film and thereafter developing the resist upper layer film with a developer to form a circuit pattern on the resist upper layer film;
   (III-6) transferring a pattern to the adhesion film and the inorganic hard mask middle layer film by dry etching with the resist upper layer film on which the circuit pattern is formed as a mask;
   (III-7) transferring a pattern to the resist underlayer film by dry etching with the inorganic hard mask middle layer film to which the pattern is transferred as a mask; and
   (III-8) processing the substrate to be processed to form a pattern on the substrate to be processed with the resist underlayer film to which the pattern is transferred as a mask, 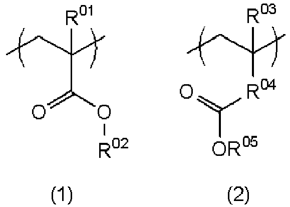 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 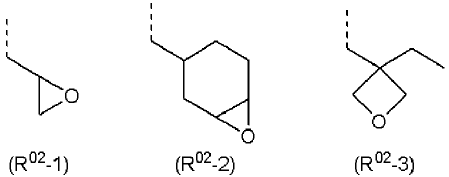 wherein a broken line represents a bond.
[4]: The pattern forming method of the above [3], wherein the inorganic hard mask middle layer film is formed by a carbon vapor deposition (CVD) method or an atomic layer deposition (ALD) method.
[5]: The pattern forming method of the above [1], [2], [3], or [4], wherein a film thickness of the adhesion film immediately below the resist upper layer film is 15 nm or less.
[6]: The pattern forming method of the above [1], [2], [3], [4], or [5], wherein the resist upper layer film is formed using the resist upper layer film forming composition comprising at least a metallic atom-containing compound and an organic solvent.
[7]: The pattern forming method of the above [6], wherein the metallic atom-containing compound comprises at least one selected from the group consisting of titanium, cobalt, copper, zinc, zirconium, lead, indium, tin, antimony, and hafnium.
[8]: The pattern forming method of the above [1], [2], [3], [4], [5], [6], or [7], wherein, as a method for forming the circuit pattern on the resist upper layer film, photolithography with a wavelength of 10 nm or more and 300 nm or less, electron beam direct drawing, nanoimprinting, or a combination thereof is used.
[9]: The pattern forming method of the above [1], [2], [3], [4], [5], [6], [7] or [8], wherein, as a development method, alkaline development or development with an organic solvent is used.
[10]: The pattern forming method of the above [1], [2], [3], [4], [5], [6], [7], [8], or [9], wherein, as the substrate to be processed, a semiconductor apparatus substrate, or the semiconductor apparatus substrate on which any one of a metal film, a metal carbide film, a metal oxide film, a metal nitride film, a metal oxide carbide film, and a metal oxide nitride film is formed is used.
[11]: The pattern forming method of the above [10], wherein, as the metal, silicon, titanium, tungsten, hafnium, zirconium, chrome, germanium, copper, silver, gold, aluminum, indium, gallium, arsenic, palladium, iron, tantalum, iridium, cobalt, manganese, molybdenum, or an alloy thereof is used.
[12]: The pattern forming method of the above [1], [2], [3], [4], [5], [6], [7], [8], [9], [10] or [11], wherein the adhesion film is formed by rotary application of the adhesion film forming composition, and thermal treatment on a substrate to which the adhesion film forming composition is applied at a temperature of 100°C or more and 300°C or less during a period of time in a range of 10 to 600 seconds.
[13]: The pattern forming method of the above [1], [2], [3], [4], [5], [6], [7], [8], [9], [10] or [11], wherein the adhesion film is formed by rotary application of the adhesion film forming composition, and thermal treatment on a substrate to which the adhesion film forming composition is applied in an atmosphere with an oxygen concentration of 0.1% or more and 21% or less.
[14]: The pattern forming method of the above [1], [2], [3], [4], [5], [6], [7], [8], [9], [10] or [11], wherein the adhesion film is formed by rotary application of the adhesion film forming composition, and thermal treatment on a substrate to which the adhesion film forming composition is applied in an atmosphere with an oxygen concentration of less than 0.1%.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(I-1) applying the adhesion film forming composition onto the substrate to be processed and thereafter performing thermal treatment to form an adhesion film;
(I-2) forming a resist upper layer film on the adhesion film using a resist upper layer film forming composition;
(I-3) performing pattern exposure on the resist upper layer film and thereafter developing the resist upper layer film with a developer to form a circuit pattern on the resist upper layer film; and
(I-4) transferring a pattern to the adhesion film and the substrate to be processed by dry etching with the resist upper layer film on which the circuit pattern is formed as a mask, 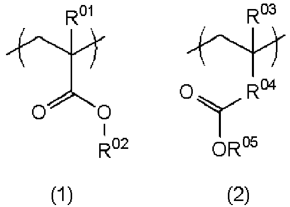 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 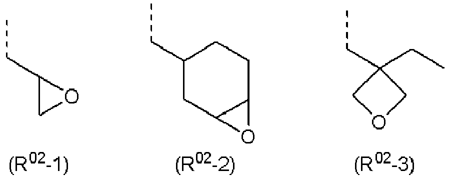 wherein a broken line represents a bond.

2. A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(II-1) forming a resist underlayer film on the substrate to be processed;
(II-2) forming a silicon-containing resist middle layer film on the resist underlayer film;
(II-3) applying the adhesion film forming composition onto the silicon-containing resist middle layer film and thereafter performing thermal treatment to form an adhesion film;
(II-4) forming a resist upper layer film on the adhesion film using a resist upper layer film forming composition;
(II-5) performing pattern exposure on the resist upper layer film and thereafter developing the resist upper layer film with a developer to form a circuit pattern on the resist upper layer film;
(II-6) transferring a pattern to the adhesion film and the silicon-containing resist middle layer film by dry etching with the resist upper layer film on which the circuit pattern is formed as a mask;
(II-7) transferring a pattern to the resist underlayer film by dry etching with the silicon-containing resist middle layer film to which the pattern is transferred as a mask; and
(II-8) processing the substrate to be processed to form a pattern on the substrate to be processed with the resist underlayer film to which the pattern is transferred as a mask, 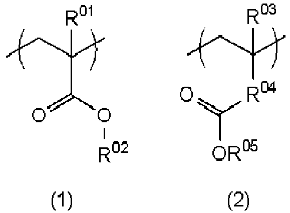 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 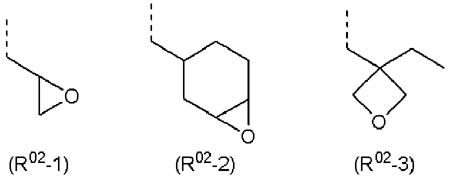 wherein a broken line represents a bond.

3. A pattern forming method for forming a pattern on a substrate to be processed using, as an adhesion film forming composition for forming an adhesion film immediately below a resist upper layer film, an adhesion film forming composition comprising (A) a polymer compound containing a repeating unit shown by the following general formula (1) and a repeating unit shown by the following general formula (2), (B) a thermal acid generator, and (C) an organic solvent, the method comprising steps of:
(III-1) forming a resist underlayer film on the substrate to be processed;
(III-2) forming, on the resist underlayer film, an inorganic hard mask middle layer film selected from the group consisting of a silicon oxide film, a silicon nitride film, and a silicon oxide nitride film;
(III-3) applying the adhesion film forming composition onto the inorganic hard mask middle layer film and thereafter performing thermal treatment to form an adhesion film;
(III-4) forming a resist upper layer film on the adhesion film using a resist upper layer film forming composition;
(III-5) performing pattern exposure on the resist upper layer film and thereafter developing the resist upper layer film with a developer to form a circuit pattern on the resist upper layer film;
(III-6) transferring a pattern to the adhesion film and the inorganic hard mask middle layer film by dry etching with the resist upper layer film on which the circuit pattern is formed as a mask;
(III-7) transferring a pattern to the resist underlayer film by dry etching with the inorganic hard mask middle layer film to which the pattern is transferred as a mask; and
(III-8) processing the substrate to be processed to form a pattern on the substrate to be processed with the resist underlayer film to which the pattern is transferred as a mask, 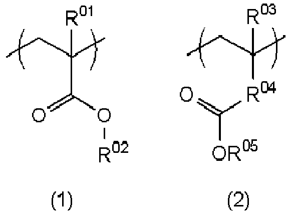 wherein R⁰¹ and R⁰³ each independently represent a hydrogen atom or a methyl group; R⁰² represents an organic group selected from the group consisting of the following formulae (R⁰²-1) to (R⁰²-3); R⁰⁴ represents a single bond or a divalent linking group containing an ester group and having 2 to 10 carbon atoms; and R⁰⁵ represents a saturated or unsaturated tertiary alkyl group having 4 to 20 carbon atoms, and optionally further contain an oxygen functional group; 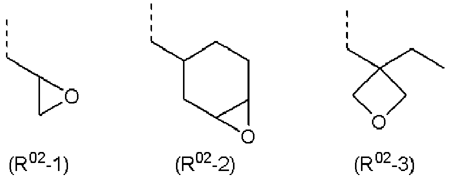 wherein a broken line represents a bond.

4. The pattern forming method according to claim 3, wherein the inorganic hard mask middle layer film is formed by a carbon vapor deposition (CVD) method or an atomic layer deposition (ALD) method.

5. The pattern forming method according to any one of claims 1 to 4, wherein a film thickness of the adhesion film immediately below the resist upper layer film is 15 nm or less.

6. The pattern forming method according to any one of claims 1 to 5, wherein the resist upper layer film is formed using the resist upper layer film forming composition comprising at least a metallic atom-containing compound and an organic solvent.

7. The pattern forming method according to claim 6, wherein the metallic atom-containing compound comprises at least one selected from the group consisting of titanium, cobalt, copper, zinc, zirconium, lead, indium, tin, antimony, and hafnium.

8. The pattern forming method according to any one of claims 1 to 7, wherein, as a method for forming the circuit pattern on the resist upper layer film, photolithography with a wavelength of 10 nm or more and 300 nm or less, electron beam direct drawing, nanoimprinting, or a combination thereof is used.

9. The pattern forming method according to any one of claims 1 to 8, wherein, as a development method, alkaline development or development with an organic solvent is used.

10. The pattern forming method according to any one of claims 1 to 9, wherein, as the substrate to be processed, a semiconductor apparatus substrate, or the semiconductor apparatus substrate on which any one of a metal film, a metal carbide film, a metal oxide film, a metal nitride film, a metal oxide carbide film, and a metal oxide nitride film is formed is used.

11. The pattern forming method according to claim 10, wherein, as the metal, silicon, titanium, tungsten, hafnium, zirconium, chrome, germanium, copper, silver, gold, aluminum, indium, gallium, arsenic, palladium, iron, tantalum, iridium, cobalt, manganese, molybdenum, or an alloy thereof is used.

12. The pattern forming method according to any one of claims 1 to 11, wherein the adhesion film is formed by rotary application of the adhesion film forming composition, and thermal treatment on a substrate to which the adhesion film forming composition is applied at a temperature of 100°C or more and 300°C or less during a period of time in a range of 10 to 600 seconds.

13. The pattern forming method according to any one of claims 1 to 11, wherein the adhesion film is formed by rotary application of the adhesion film forming composition, and thermal treatment on a substrate to which the adhesion film forming composition is applied in an atmosphere with an oxygen concentration of 0.1% or more and 21% or less.

14. The pattern forming method according to any one of claims 1 to 11, wherein the adhesion film is formed by rotary application of the adhesion film forming composition, and thermal treatment on a substrate to which the adhesion film forming composition is applied in an atmosphere with an oxygen concentration of less than 0.1%.
